# EUROPEAN PATENT APPLICATION

(11) **EP 3 988 106 A1**
(43) Date of publication of application: **27.04.2022**
(21) Application number: 21196509.0
(22) Date of filing: 11.03.2016
(51) Int. Cl.: A61K 35/17, A61P 35/00, A61P 37/06

(54) **IL-10-PRODUCING CD4+ T CELLS AND USES THEREOF**

(30) Priority: 13.03.2015 EP 15159075
(62) Divisional of application: 16710702.8
(71) Applicant: Ospedale San Raffaele S.r.l., 20132 Milano (IT); Fondazione Centro San Raffaele, 20132 Milano (IT); Fondazione Telethon, 00185 Roma (IT)
(72) Inventor: Gregori, Silvia Adriana, 20132 Milano (IT); Roncarolo, Maria Grazia, 20132 Milano (IT)
(74) Representative: Ghirardi, Valeria

(57) **Abstract**

The present invention relates to a CD4⁺ T cell that produces high levels of IL-10 for use in the treatment and/or prevention of a tumor that expresses CD13, HLA-class I and CD54 and/or for use in inducing Graft versus tumour (GvT). The present invention relates also to a composition comprising said cell and to a method to select a subject to be treated with said cell.

SEQUENCE LISTING

<110>  Ospedale San Raffaele S.r.l.
       Fondazione Centro San Raffaele
       Fondazione Telethon

<120>  IL-10-producing CD4+ T cells and uses thereof

<130>  BE 148897

<150>  EP15159075.9
<151>  2015-03-13

<160>  1

<170>  PatentIn version 3.5

<210>  1
<211>  20
<212>  DNA
<213>  Artificial Sequence

<220>
<223>  synthetic

<400>  1
gagtagcgcg agcacagcta                                                   20

## Description

### Technical field

The present invention relates to a CD4⁺ T cell that produces high levels of IL-10 for use in the treatment and/or prevention of a tumor that expresses CD13, HLA-class I and CD54 and/or for use in inducing Graft versus tumour (GvT). The present invention relates also to a composition comprising said cell and to a method to select a subject to be treated with said cell.

### Background art

T regulatory cells (Tregs) are a fundamental component of the healthy immune system since they play a pivotal role in promoting and maintaining tolerance. Over the years, several types of Tregs have been identified and, to date, the best characterized are the forkhead box P3 (FOXP3)-expressing Tregs (CD25⁺ Tregs) ¹ and the T regulatory type 1 (Tr1) cells ². Tr1 cells are induced in the periphery upon chronic antigen(Ag) stimulation in the presence of IL-10 ², and are characterized by the co-expression of CD49b and LAG-3 ³ and the ability to secrete IL-10, Transforming Growth Factor (TGF)-β, variable amounts of IFN-γ, and low levels of IL-2, and minimal amounts of IL-4 and IL-17 ²⁻⁴. Tr1 cells suppress T-cell responses primarily *via* the secretion of IL-10 and TGF-β ^{2,5} and by the specific killing of myeloid antigen-presenting cells through the release of Granzyme B (GzB) and perforin ⁶. Tr1 cells are induced *in vitro* when T cells are activated in the presence of recombinant human IL-10 or tolerogenic dendritic cells (DC-10) that secrete high amounts of IL-10 and express immunoglobulin-like transcript-4 (ILT4) and HLA-G ^{7,8}.

In the past decade much effort has been dedicated to develop suitable methods for the *in vitro* induction/expansion of CD25⁺ Tregs and of Tr1 cells for Treg-based cell therapy to promote or restore tolerance in T-cell mediated diseases. Treg-based cell therapy has been extensively tested in pre-clinical models of Graft-versus-Host-Disease (GvHD) ⁹⁻¹¹ and humanized mouse models of xeno-GvHD ¹². Proof-of-principle clinical trials in allogeneic hematopoietic stem cell transplantation (allo-HSCT) demonstrated the safety of CD25⁺ Treg-based cell therapy ¹³⁻¹⁷. Freshly isolated ^{14,15,17} or *in vitro* expanded polyclonal CD25⁺ Tregs ^{13,16} were infused after allo-HSCT or haploidentical HSCT for oncological malignancies to prevent GvHD. In these studies a reduction of GvHD was observed compared to historical controls. Recently, it has been shown that in CD25⁺ Treg-treated patients the cumulative incidence of relapse was significantly lower than in controls ¹⁷. The inventors pursued a donor-patient tailored approach using host-specific IL-10-anergized donor T cells (IL-10 DLI), containing Tr1 cells as Treg-based therapy. IL-10 DLI was obtained *in vitro* with alloAg stimulation in the presence of exogenous IL-10 or DC-10. The inventors demonstrated the safety and feasibility of Tr1 cell-infusion in a clinical trial aimed at providing immune reconstitution in the absence of severe GvHD in hematological cancer patients undergoing haploidentical HSCT ¹⁸. Although a small cohort of patients was treated, results demonstrated that after infusion of IL-10 DLI only mild GvHD (grade II or III, responsive to therapy) was observed and a tolerance signature was achieved. Furthermore, the treatment accelerated immune reconstitution after transplantation, and correlated with long-lasting disease remission¹⁸. A major difference between the CD25⁺ Treg-based trials and the IL-10 DLI trial is that, in the formers, a pool of polyclonal non-Ag-specific cells was administered, whereas the inventors used a cell product containing *in vitro* primed donor-derived host-specific Tr1 cells.

Andolfi et al. discloses the use of a bidirectional lentiviral vector (LV) encoding for human IL-10 and the marker gene, green fluorescent protein (GFP), which are independently co-expressed to generate CD4^{LV-IL-10} cells. CD4^{LV-IL-10} cells displayed typical Tr1 features: the anergic phenotype, the IL-10- and TGF-β-dependent suppression of allogeneic T-cell responses, the ability to suppress in a cell-to-cell contact independent manner *in vitro.* CD4^{LV-IL-10} cells were able to control xeno graft-versus-host disease (GvHD), demonstrating their suppressive function *in vivo* ¹⁸.

Despite recent advances in the establishment of protocols of Tr1-based immunotherapy with *in vitro* induced alloAg-specific IL-10-anergized T cells, the resulting populations still contain contaminants that could potentially limit the *in vivo* efficacy of Tr1 cells to modulate T cell-mediated responses. Therefore, there is the need for a pure cell population expressing IL-10 for use in therapy.

### Summary of the invention

In the present invention, a CD4+ T cell that produces high levels of IL-10 was generated. In particular, an homogenous IL-10-engineered CD4⁺ T (CD4^{IL-10}) cell population was generated by transducing human CD4+ T cells with a bidirectional lentiviral vector (LV) encoding for human IL-10 and ΔNGFR, as clinical grade marker gene, leading to a constitutive over expression of IL-10. Surprisingly the CD4^{IL-10} cell population of the invention was able to eliminate tumor, but maintained the intrinsic characteristic, Tr1-like, to prevent xeno-GvHD. Surprisingly, the CD4^{IL-10} cell population of the invention kills tumors (or target cells) expressing CD13. The expression of CD13 on the tumor or target cells is determinant for the anti-tumoral activity of the CD4^{IL-10} cell population. Moreover, the killing activity of CD4^{IL-10} of the invention requires the presence of CD13, HLA-class I and CD54 on the tumor. Therefore, the adoptive transfer of CD4^{IL-10} cells of the invention mediates *in vivo* potent anti-tumor effect, preferably an anti-leukemia effect (Graft versus Leukemia, GvL), and prevents xeno-GvHD without compromising the GvL effect mediated by HSCT.

Similarly to the polyclonal CD4^{IL-10} T cells described above, allo-specific CD4^{IL-10} T cells are also capable of eliminating CD13⁺ tumor cell lines in an HLA-1 dependent manner.

CD4^{IL-10} cells of the present invention homogenously express GzB, are CD18⁺, which in association with CD11a forms LFA-1, CD2⁺, and CD226⁺. Moreover, CD4^{IL-10} cells of the present invention acquired the ability to eliminate target cells, such as tumor cells for instance primary myeloid cells, such as primary leukemic blasts. The inventors demonstrate that anti-leukemic activity of CD4^{IL-10} cells is specific for myeloid cells and requires the presence of HLA-class I on the tumor. The inventors identify HLA-class I CD13, CD54, and optionally CD112 as biomarkers of CD4^{IL-10} cell anti-leukemic activity. Moreover, the inventors provide evidences that adoptive transfer of CD4^{IL-10} cells mediate *in vivo* potent anti-myeloid tumor and anti-leukemic effects and prevent xeno-GvHD without compromising the anti-leukemic effect mediated by allogeneic T cells.

In summary the inventors showed that CD4^{IL-10} cells i) specifically killed target cells, in particular myeloid cells; ii) mediate anti-tumor (GvT) or anti-leukemic (GvL) effects, iii) allow allogeneic T cells to maintain their anti-leukemic (GvL) effect; and iv) maintain the ability to inhibit GvHD. In the contest of solid tumors the ability of immunotherapy with CD4^{IL-10} cells to eliminate tumor cells is termed graft-versus-tumor (GvT), whereas in the contest of hematological diseases the anti-tumor effect mediated by immunotherapy with CD4^{IL-10} cells or allogeneic T cells is named Graft-versus-Leukemia (GvL).

Based on the findings, CD4^{IL-10} cells of the present invention prevent GvHD while at the same time preserve GvL in patients affected by a variety of hematological malignancies who receive allo-HSCT. In addition, the ability of CD4^{IL-10} cells to eliminate myeloid leukemia in an alloAg-independent but HLA class I-dependent manner renders them an interesting therapeutic approach to limit, and possibly to overcome, leukemia relapse caused by the loss of shared HLA alleles after haploidentical-HSCT, or matched unrelated HSCT. Moreover, CD4^{IL-10} cells mediate anti-leukemic effects in an alloAg-independent manner, rendering possible the use of autologous or even third party cells as immunotherapy. Furthermore, CD4^{IL-10} cells might be used as anti-tumor cells in the contest of other malignancies mediated by aberrant myeloid cells, such as in extramedullary manifestations (EM) of AML, which include myeloid sarcoma and leukemia cutis. Thus far, the treatment for EM is chemotherapy. Notably, EM is often a form of relapse after allo-HSCT. Finally, being able to eliminate also non-transformed myeloid cells (i.e. monocytes and myeloid DC), CD4^{IL-10} cells may also be used as adjuvant therapy in other malignancies, such as in solid tumors, where tumor-associated myeloid cells are known to play a critical role in promoting tumor neo-vascularization.

The inventors generated a homogeneous population of human IL-10 producing CD4⁺ T (CD4^{IL-10}) cells by lentiviral vector-mediated gene transfer. CD4^{IL-10} cells of the present invention i) specifically kill target cells that express CD13, CD54 and HLA-class I in particular leukemic cells and primary blasts. The target cells may also express further markers such as CD112, CD58 or CD155, ii) mediate anti-leukemic and anti-tumor effects *in vivo,* and iii) preserve GvL mediated by allogeneic T cells.

Overall, immunotherapy with CD4^{IL-10} cells represents an innovative tool to prevent GvHD in patients affected by a variety of hematological malignancies who receive allo-HSCT. In addition, it opens new perspectives also in the contest of other malignancies mediated by aberrant myeloid cells.

In particular, the present invention relates to some specific applications of CD4^{IL-10} cells:
1) They target cells e.g. tumor cells, in particular leukemic blasts, expressing CD13, CD54 and HLA-I, optionally CD112, leading to a method to select patient to be treated with the CD4^{IL-10} cells.
2) CD4^{IL-10} cells adoptively transferred *in vivo* mediate GvL but do not hamper the GvL effect of human allogeneic T cells.
3) CD4^{IL-10} cells inhibit the expansion of allogeneic T cells in the peripheral tissues, preventing GvHD.
4) Selection of patient and design of ad hoc therapeutic protocol: CD4^{IL-10} cells may be used not only for preventing GvHD after allogeneic HSCT but also as immunotherapy for selectively eliminate blasts in case of relapse of the disease.

The surprising and remarkable effect of the CD4^{IL-10} cells of the present invention resides in the fact that not only such cells are able to suppress tumor (such as hematological tumors) but they do not induce GvHD, as allo-HSCT does. Moreover the CD4^{IL-10} cells of the present invention do not inhibit GvL mediated by allo-HSCT. All together these data demonstrate the superior and advantageous properties of the CD4^{IL-10} cells of the present invention for the tumors and for the treatment and/or prevention of GvHD preserving at the same time GvT and/or GvL after allo-HSCT to cure myeloid malignancies. Therefore the present invention provides a CD4⁺ T cell that produces high levels of IL-10 for use in the treatment and/or prevention of a tumor, wherein said tumor expresses CD13, HLA-class I and CD54. Preferably said cell is modified to produce high levels of IL-10. Preferably said cell is genetically modified to produce IL-10. Preferably said cell expresses CD18 and/or CD2 and/or CD226. Still preferably said cell is CD226⁺⁺, i.e expresses high levels of CD226. Preferably said cell expresses granzyme B (GzB).

Preferably said cell prevents GvHD. Preferably said cell induces Graft versus Tumour (GvT) and/or induces Graft versus leukemia (GvL). Preferably said cell is autologous, heterologous, polyclonal or allo-specific. A preferred cell is autologous and polyclonal or heterologous and polyclonal.

Preferably said tumor further expresses at least one marker selected from the group consisting of: CD112, CD58. Preferably said tumor further expresses CD155. Preferably the tumor is a solid or hematological tumor. Preferably the tumor is leukemic or a myeloid tumor.

Preferably the tumor is mediated by a cell selected from the group consisting of: macrophage, monocyte, granulocyte, erythrocyte, thrombocyte, mast cell, B cell, T cell, NK cell, dendritic cell, Kupffer cell, microglial cell and plasma cell. Preferably the solid tumor or the hematological tumor is selected from a cell of the group consisting of: Adrenal Cancer, Anal Cancer, Bile Duct Cancer, Bladder Cancer, Bone Cancer, Brain/CNS Tumors In Adults, Brain/CNS Tumors In Children, Breast Cancer, Breast Cancer In Men, Cancer of Unknown Primary, Castleman Disease, Cervical Cancer, Colon/Rectum Cancer, Endometrial Cancer, Esophagus Cancer, Ewing Family Of Tumors, Eye Cancer, Gallbladder Cancer, Gastrointestinal Carcinoid Tumors, Gastrointestinal Stromal Tumor (GIST), Gestational Trophoblastic Disease, Hodgkin Disease, Kaposi Sarcoma, Kidney Cancer, Laryngeal and Hypopharyngeal Cancer, Leukemia, Acute Lymphocytic (ALL), Acute Myeloid (AML, including myeloid sarcoma and leukemia cutis), Chronic Lymphocytic (CLL), Chronic Myeloid (CML) Leukemia, Chronic Myelomonocytic (CMML), Leukemia in Children, Liver Cancer, Lung Cancer, Lung Cancer with Non-Small Cell, Lung Cancer with Small Cell, Lung Carcinoid Tumor, Lymphoma, Lymphoma of the Skin, Malignant Mesothelioma, Multiple Myeloma, Myelodysplastic Syndrome, Nasal Cavity and Paranasal Sinus Cancer, Nasopharyngeal Cancer, Neuroblastoma, Non-Hodgkin Lymphoma, Non-Hodgkin Lymphoma In Children, Oral Cavity and Oropharyngeal Cancer, Osteosarcoma, Ovarian Cancer, Pancreatic Cancer, Penile Cancer, Pituitary Tumors, Prostate Cancer, Retinoblastoma, Rhabdomyosarcoma, Salivary Gland Cancer, Sarcoma - Adult Soft Tissue Cancer, Skin Cancer, Skin Cancer - Basal and Squamous Cell, Skin Cancer - Melanoma, Skin Cancer - Merkel Cell, Small Intestine Cancer, Stomach Cancer, Testicular Cancer, Thymus Cancer, Thyroid Cancer, Uterine Sarcoma, Vaginal Cancer, Vulvar Cancer, Waldenstrom Macroglobulinemia, Wilms Tumor.

Preferably the tumor is refractory to a therapeutic intervention. Preferably said cell is used in combination with a therapeutic intervention. The combination may be simultaneous or performed at different times. Preferably the therapeutic intervention is selected from the group consisting of: chemotherapy, radiotherapy, allo-HSCT, blood transfusion, blood marrow transplant.

The invention also provides a CD4⁺ T cell that produces high levels of IL-10 for use in the treatment and/or prevention of leukemia relapse.

The invention also provides a CD4⁺ T cell that produces high levels of IL-10 for use in a method to induce Graft versus tumour (GvT). Preferably said cell is modified to produce high levels of IL-10. Preferably said cell is genetically modified to produce IL-10.

The invention also provides a composition comprising a CD4⁺ T cell as defined above and proper excipients for use in the treatment and/or prevention of a tumor wherein said tumor expresses CD13, HLA-class I and CD54 or for use in the treatment and/or prevention of leukemia relapse or for use in a method to induce Graft versus tumour (GvT).

Preferably the composition further comprises a therapeutic agent.

The invention also provides a method to select a subject to be treated with a CD4⁺ T cell that produces high level of IL-10 comprising detecting the presence of a cell that expresses CD13, HLA-class I and CD54 in a biological sample obtained from the subject, wherein if the presence of the cell that expresses CD13, HLA-class I and CD54 is detected, the subject is selected to be treated with said CD4⁺ T cell. Preferably the cell further expresses CD112 and/or CD58. Preferably the presence of CD13, HLA-class I, CD54, and CD112 is detected.

Preferably the cell further expresses CD155.

The invention also provides a kit for use in the method as defined above comprising means to detect the presence of a cell that expresses at least CD13, HLA-class I and CD54 in a biological sample.

In the present invention any CD4+ cell that produces high levels of IL-10 (also named CD4^{IL-10}) is contemplated. Preferably the cell constitutively produces high levels of IL-10. Such cell may be obtained by any genetic modifications, cell fusion or any other means known in the art. A CD4+ T cell that produces high levels of IL-10 may be generated by any means known to the skilled person in the art. For instance a pure population of IL-10-producing cells can be obtained by selection of CD49b⁺LAG-3⁺ T cells from induced alloAG-specific IL-10-anergized T cells (as described in WO2013192215).

Said cell produce IL-10 is a CD4+ T cell that constitutively produces, overexpresses or expresses high levels of IL-10 in respect to a reference cell. In particular, the cell secretes at least 1 ng/ml of IL-10 as determined according to known methods in the art, such as described in the material and method section.

In the present invention the CD4+ cell genetically modified to produce IL-10 is a CD4+ T cell that constitutively produces, overexpresses or expresses high levels of IL-10 in respect to a reference cell. In particular, the cell secretes at least 1 ng/ml of IL-10 as determined according to known methods in the art, such as described in the material and method section.

The CD4+ T cell may also produce or express high levels of IFN-γ in respect of a reference cell. In particular, the cell expresses at least 1 ng/ml of IFNγ as determined according to known methods in the art, such as described in the material and method section.

In the present invention a reference cell may be a CD4⁺ cell transduced with a lentivirus for the expression of GFP (CD4^{GFP} cells), as described herein.

In the present invention the subject to be treated is affected by a tumor and may receive autologous or heterologous polyclonal CD4+ T cells that produce high level of IL-10.

In the present invention a polyclonal CD4+ T cell means a CD4+ T cell isolated from peripheral blood or cord blood. Said polyclonal CD4+ T cell may be autologous (when it has been obtained from the patient to be treated) or heterologous (when it has been obtained from a subject that is not the patient to be treated).

In an alternative therapeutic situation, the subject is affected by a tumour and receive an allogenic hematopietic stem cell transplantation (allo-HSCT) in such a case, donor CD4+ T cells are contacted with patient antigen-presenting cells (monocytes or dendritic cells) , generating allo-specific CD4+ T cells that are then modified to produce high level of IL-10 (allo-CD4^{IL-10} cell).

A Tr1-like cell is a cell that recapitulated the features of a Tr1 cell: Tr1 cells suppress T-cell responses primarily *via* the secretion of IL-10 and TGF-β and by the specific killing of myeloid antigen-presenting cells through the release of Granzyme B (GzB) and perforin.

In the present invention the CD4+ T cells genetically modified produce constitutively high levels of IL-10 and may be use as adjuvant therapy in protocols aims at preventing GvHD while allowing to maintain GvL after allo-HSCT.

In the present invention Graft-versus-leukemia (GvL) is a major component of the overall beneficial effects of allogeneic bone marrow transplantation (BMT) in the treatment of leukemia. The term graft-versus-leukemia (GvL) is used to describe the immune-mediated response, which conserves a state of continued remission of a hematological malignancy following allogeneic marrow stem cell transplants. GvL effect after allogeneic bone marrow transplantation (BMT) is well accepted. In GvL reactions, the allo-response suppresses residual leukemia.

Graft versus tumor effect (GvT) appears after allogeneic hematopoietic stem cell transplantation (HSCT). The graft contains donor T lymphocytes that are beneficial for recipient. Donor T-cells eliminate malignant residual host T-cells (GvL) or eliminates diverse kinds of tumors. GvT might develop after recognizing tumor-specific or recipient-specific alloantigens. It could lead to remission or immune control of hematologic malignancies. This effect applies in myeloma and lymphoid leukemias, lymphoma, multiple myeloma and possibly breast cancer.

In the present invention allo-specific immunotherapy means cell therapy with T cells that have been primed/stimulated with cells isolated or generated from an allogeniec donor. In case of allo-HSCT, it means T cells from the HSCT donor that have been primed/stimulated with cells isolated from the recipient (patient) who will be treated with HSCT

The CD4+ T cell genetically modified to produce constitutively high levels of IL-10 of the present invention may be used alone or in combination with other therapeutic intervention such as radiotherapy, chemotherapy, immunosuppressant and immunomodulatory therapies . Chemotherapy may include Abitrexate (Methotrexate Injection), Abraxane (Paclitaxel Injection), Adcetris (Brentuximab Vedotin Injection), Adriamycin (Doxorubicin), Adrucil Injection (5-FU (fluorouracil)), Afinitor (Everolimus) , Afinitor Disperz (Everolimus) , Alimta (PEMETREXED), Alkeran Injection (Melphalan Injection), Alkeran Tablets (Melphalan), Aredia (Pamidronate), Arimidex (Anastrozole), Aromasin (Exemestane), Arranon (Nelarabine), Arzerra (Ofatumumab Injection), Avastin (Bevacizumab), Bexxar (Tositumomab), BiCNU (Carmustine), Blenoxane (Bleomycin), Bosulif (Bosutinib), Busulfex Injection (Busulfan Injection), Campath (Alemtuzumab), Camptosar (Irinotecan), Caprelsa (Vandetanib), Casodex (Bicalutamide), CeeNU (Lomustine), CeeNU Dose Pack (Lomustine), Cerubidine (Daunorubicin), Clolar (Clofarabine Injection), Cometriq (Cabozantinib), Cosmegen (Dactinomycin), CytosarU (Cytarabine), Cytoxan (Cytoxan), Cytoxan Injection (Cyclophosphamide Injection), Dacogen (Decitabine), DaunoXome (Daunorubicin Lipid Complex Injection), Decadron (Dexamethasone), DepoCyt (Cytarabine Lipid Complex Injection), Dexamethasone Intensol (Dexamethasone), Dexpak Taperpak (Dexamethasone), Docefrez (Docetaxel), Doxil (Doxorubicin Lipid Complex Injection), Droxia (Hydroxyurea), DTIC (Decarbazine), Eligard (Leuprolide), Ellence (Ellence (epirubicin)), Eloxatin (Eloxatin (oxaliplatin)), Elspar (Asparaginase), Emcyt (Estramustine), Erbitux (Cetuximab), Erivedge (Vismodegib), Erwinaze (Asparaginase Erwinia chrysanthemi), Ethyol (Amifostine), Etopophos (Etoposide Injection), Eulexin (Flutamide), Fareston (Toremifene), Faslodex (Fulvestrant), Femara (Letrozole), Firmagon (Degarelix Injection), Fludara (Fludarabine), Folex (Methotrexate Injection), Folotyn (Pralatrexate Injection), FUDR (FUDR (floxuridine)), Gemzar (Gemcitabine), Gilotrif (Afatinib), Gleevec (Imatinib Mesylate), Gliadel Wafer (Carmustine wafer), Halaven (Eribulin Injection), Herceptin (Trastuzumab), Hexalen (Altretamine), Hycamtin (Topotecan), Hycamtin (Topotecan), Hydrea (Hydroxyurea), Iclusig (Ponatinib), Idamycin PFS (Idarubicin), Ifex (Ifosfamide), Inlyta (Axitinib), Intron A alfab (Interferon alfa-2a), Iressa (Gefitinib), Istodax (Romidepsin Injection), Ixempra (Ixabepilone Injection), Jakafi (Ruxolitinib), Jevtana (Cabazitaxel Injection), Kadcyla (Ado-trastuzumab Emtansine), Kyprolis (Carfilzomib), Leukeran (Chlorambucil), Leukine (Sargramostim), Leustatin (Cladribine), Lupron (Leuprolide), Lupron Depot (Leuprolide), Lupron DepotPED (Leuprolide), Lysodren (Mitotane), Marqibo Kit (Vincristine Lipid Complex Injection), Matulane (Procarbazine), Megace (Megestrol), Mekinist (Trametinib), Mesnex (Mesna), Mesnex (Mesna Injection), Metastron (Strontium-89 Chloride), Mexate (Methotrexate Injection), Mustargen (Mechlorethamine), Mutamycin (Mitomycin), Myleran (Busulfan), Mylotarg (Gemtuzumab Ozogamicin), Navelbine (Vinorelbine), Neosar Injection (Cyclophosphamide Injection), Neulasta (filgrastim), Neulasta (pegfilgrastim), Neupogen (filgrastim), Nexavar (Sorafenib), Nilandron (Nilandron (nilutamide)), Nipent (Pentostatin), Nolvadex (Tamoxifen), Novantrone (Mitoxantrone), Oncaspar (Pegaspargase), Oncovin (Vincristine), Ontak (Denileukin Diftitox), Onxol (Paclitaxel Injection), Panretin (Alitretinoin), Paraplatin (Carboplatin), Perjeta (Pertuzumab Injection), Platinol (Cisplatin), Platinol (Cisplatin Injection), PlatinolAQ (Cisplatin), PlatinolAQ (Cisplatin Injection), Pomalyst (Pomalidomide), Prednisone Intensol (Prednisone), Proleukin (Aldesleukin), Purinethol (Mercaptopurine), Reclast (Zoledronic acid), Revlimid (Lenalidomide), Rheumatrex (Methotrexate), Rituxan (Rituximab), RoferonA alfaa (Interferon alfa-2a), Rubex (Doxorubicin), Sandostatin (Octreotide), Sandostatin LAR Depot (Octreotide), Soltamox (Tamoxifen), Sprycel (Dasatinib), Sterapred (Prednisone), Sterapred DS (Prednisone), Stivarga (Regorafenib), Supprelin LA (Histrelin Implant), Sutent (Sunitinib), Sylatron (Peginterferon Alfa-2b Injection (Sylatron)), Synribo (Omacetaxine Injection), Tabloid (Thioguanine), Taflinar (Dabrafenib), Tarceva (Erlotinib), Targretin Capsules (Bexarotene), Tasigna (Decarbazine), Taxol (Paclitaxel Injection), Taxotere (Docetaxel), Temodar (Temozolomide), Temodar (Temozolomide Injection), Tepadina (Thiotepa), Thalomid (Thalidomide), TheraCys BCG (BCG), Thioplex (Thiotepa), TICE BCG (BCG), Toposar (Etoposide Injection), Torisel (Temsirolimus), Treanda (Bendamustine hydrochloride), Trelstar (Triptorelin Injection), Trexall (Methotrexate), Trisenox (Arsenic trioxide), Tykerb (Iapatinib), Valstar (Valrubicin Intravesical), Vantas (Histrelin Implant), Vectibix (Panitumumab), Velban (Vinblastine), Velcade (Bortezomib), Vepesid (Etoposide), Vepesid (Etoposide Injection), Vesanoid (Tretinoin), Vidaza (Azacitidine), Vincasar PFS (Vincristine), Vincrex (Vincristine), Votrient (Pazopanib), Vumon (Teniposide), Wellcovorin IV (Leucovorin Injection), Xalkori (Crizotinib), Xeloda (Capecitabine), Xtandi (Enzalutamide), Yervoy (Ipilimumab Injection), Zaltrap (Ziv-aflibercept Injection), Zanosar (Streptozocin), Zelboraf (Vemurafenib), Zevalin (Ibritumomab Tiuxetan), Zoladex (Goserelin), Zolinza (Vorinostat), Zometa (Zoledronic acid), Zortress (Everolimus), Zytiga (Abiraterone), Nimotuzumab and immune checkpoint inhibitors such as nivolumab, pembrolizumab/MK-3475, pidilizumab and AMP-224 targeting PD-1; and BMS-935559, MEDI4736, MPDL3280A and MSB0010718C targeting PD-L1 and those targeting CTLA-4 such as ipilimumab.

Radiotherapy means the use of radiation, usually X-rays, to treat illness. X-rays were discovered in 1895 and since then radiation has been used in medicine for diagnosis and investigation (X-rays) and treatment (radiotherapy). Radiotherapy may be from outside the body as external radiotherapy, using X-rays, cobalt irradiation, electrons, and more rarely other particles such as protons. It may also be from within the body as internal radiotherapy, which uses radioactive metals or liquids (isotopes) to treat cancer.

The CD4+ T cell of the present invention may be used in an amount that can be easily determined by the skilled person in the art according to body weight and known other factors. In particular, 10⁴ to 10⁸ cells/kg may be administered. Preferably 10⁶ cells/kg are used.

The CD4+ T cell genetically modified to produce constitutively high levels of IL-10 of the present invention may be used with a single or multiple administrations. For instance the CD4+ T cell producing high levels of IL-10 of the invention, in particular genetically modified, may be administered according to different schedules comprising: every day, every 7 days or every 14 or 21 days, every month.

The CD4+ T cell of the present invention may be administered according to different administration routes comprising systemically, subcutaneously, intraperitoneally. Typically the cell is administered as it is, within a saline or physiological solution which may contain 2-20 % , preferably 7 % human serum albumin.

In the case of solid tumor, the CD4+ T cell of the present invention may act on cells surrounding the tumor such as monocytes/macrophages, Kupffer cells, microglia.

In the case of hematological tumor, the CD4+ T cell of the present invention may act on the tumor cell itself, for instance on leukemia blasts expressing CD13, CD54, HLA-class I, and optionally CD112.

In the present invention a target cell is any cell type that expresses the cell surface marker CD13, CD54 and HLA-class I. A target cell comprises a fibroblast, a mesenchymal cell or a myeloid cell.

In the present invention a solid tumour is a neoplasm (new growth of cells) or lesion (damage of anatomic structures or disturbance of physiological functions) formed by an abnormal growth of body tissue cells other than blood, bone marrow or lymphatic cells. A solid tumour consists of an abnormal mass of cells which may stem from different tissue types such as liver, colon, breast, or lung, and which initially grows in the organ of its cellular origin. However, such cancers may spread to other organs through metastatic tumor growth in advanced stages of the disease.

In the present invention a hematological tumor is a cancer type affecting blood, bone marrow, and lymph nodes. Hematological tumours may derive from either of the two major blood cell lineages: myeloid and lymphoid cell lines. The myeloid cell line normally produces granulocytes, erythrocytes, thrombocytes, macrophages, and mast cells, whereas the lymphoid cell line produces B, T, NK and plasma cells. Lymphomas (e.g. Hodgkin's Lymphoma), lymphocytic leukemias, and myeloma are derived from the lymphoid line, while acute and chronic myelogenous leukemia (AML, CML), myelodysplastic syndromes and myeloproliferative diseases are myeloid in origin. As blood, bone marrow, and lymph nodes are intimately connected through the immune system, a disease affecting one haematological system may affect the two others as well.

In the present invention the tumor to be treated may be refractory or resistant to a therapeutic intervention. The tendency of malignant cells to acquire mutations that allow them to resist the effects of antineoplastic drugs is an important factor limiting the effectiveness of chemotherapy. Tumors that are heterogeneous mixtures of chemo sensitive and chemo resistant cells may initially appear to respond to treatment, but then relapse as the chemo sensitive cells are killed off and the drug resistant cells become predominant.

The most common mechanism for this is over-expression of specialized proteins embedded in the plasma membrane called glycoprotein that actively "pump" drugs out of the cell before they can exert their pharmacological effect. Since this mechanism is not drug-specific (i.e., it works on any potentially toxic molecule) it can make a tumor resistant to many drugs - even drugs to which it has not been previously exposed. This important phenomenon is called "multiple drug resistance".

Items:
1. A CD4⁺ T cell that produces high levels of IL-10 for use in the treatment and/or prevention of a tumor, wherein said tumor expresses CD13, HLA-class I and CD54.
2. The CD4⁺ T cell for use according to item 1 wherein said cell is modified to produce high levels of IL-10.
3. The CD4⁺ T cell for use according to item 1 or 2 wherein said cell is genetically modified to produce IL-10.
4. The CD4⁺ T cell for use according to any one of previous items wherein said cell expresses CD18 and/or CD2 and/or CD226.
5. The CD4⁺ T cell for use according to any one of previous items wherein said cell prevents GvHD.
6. The CD4⁺ T cell for use according to any one of previous items wherein said cell induces Graft versus Tumour (GvT) and/or induces Graft versus leukemia (GvL).
7. The CD4⁺ T cell for use according to any one of previous items wherein said cell is autologous, heterologous, polyclonal or allo-specific.
8. The CD4⁺ T cell for use according to any one of previous items wherein said tumor further expresses at least one marker selected from the group consisting of: CD112, CD58.
9. The CD4⁺ T cell for use according to any one of previous items wherein said tumor further expresses CD155.
10. The CD4⁺ T cell for use according to any one of previous items wherein the tumor is a solid or hematological tumor.
11. The CD4⁺ T cell for use according to item 9 wherein said tumor is leukemic or a myeloid tumor.
12. The CD4⁺ T cell for use according to item 10 wherein the tumor is mediated by a cell selected from the group consisting of: macrophage, monocyte, granulocyte, erythrocyte, thrombocyte, mast cell, B cell, T cell, NK cell, dendritic cell, Kupffer cell, microglial cell and plasma cell.
13. The CD4⁺ T cell for use according to any one of item 11 to 12 wherein the solid tumor or the hematological tumor is selected from a cell of the group consisting of: Adrenal Cancer, Anal Cancer, Bile Duct Cancer, Bladder Cancer, Bone Cancer, Brain/CNS Tumors In Adults, Brain/CNS Tumors In Children, Breast Cancer, Breast Cancer In Men, Cancer of Unknown Primary, Castleman Disease, Cervical Cancer, Colon/Rectum Cancer, Endometrial Cancer, Esophagus Cancer, Ewing Family Of Tumors, Eye Cancer, Gallbladder Cancer, Gastrointestinal Carcinoid Tumors, Gastrointestinal Stromal Tumor (GIST), Gestational Trophoblastic Disease, Hodgkin Disease, Kaposi Sarcoma, Kidney Cancer, Laryngeal and Hypopharyngeal Cancer, Leukemia, Acute Lymphocytic (ALL), Acute Myeloid (AML, including myeloid sarcoma and leukemia cutis), Chronic Lymphocytic (CLL), Chronic Myeloid (CML) Leukemia, Chronic Myelomonocytic (CMML), Leukemia in Children, Liver Cancer, Lung Cancer, Lung Cancer with Non-Small Cell, Lung Cancer with Small Cell, Lung Carcinoid Tumor, Lymphoma, Lymphoma of the Skin, Malignant Mesothelioma, Multiple Myeloma, Myelodysplastic Syndrome, Nasal Cavity and Paranasal Sinus Cancer, Nasopharyngeal Cancer, Neuroblastoma, Non-Hodgkin Lymphoma, Non-Hodgkin Lymphoma In Children, Oral Cavity and Oropharyngeal Cancer, Osteosarcoma, Ovarian Cancer, Pancreatic Cancer, Penile Cancer, Pituitary Tumors, Prostate Cancer, Retinoblastoma, Rhabdomyosarcoma, Salivary Gland Cancer, Sarcoma - Adult Soft Tissue Cancer, Skin Cancer, Skin Cancer - Basal and Squamous Cell, Skin Cancer - Melanoma, Skin Cancer - Merkel Cell, Small Intestine Cancer, Stomach Cancer, Testicular Cancer, Thymus Cancer, Thyroid Cancer, Uterine Sarcoma, Vaginal Cancer, Vulvar Cancer, Waldenstrom Macroglobulinemia, Wilms Tumor.
14. The CD4⁺ T cell for use according to any one of previous items wherein the tumor is refractory to a therapeutic intervention.
15. The CD4⁺ T cell for use according to any one of previous items wherein said cell is used in combination with a therapeutic intervention.
16. The CD4⁺ T cell for use according to item 14 or 15 wherein the therapeutic intervention is selected from the group consisting of: chemotherapy, radiotherapy, allo-HSCT, blood transfusion, blood marrow transplant.
17. A CD4⁺ T cell that produces high levels of IL-10 for use in the treatment and/or prevention of leukemia relapse.
18. A CD4⁺ T cell that produces high levels of IL-10 for use in a method to induce Graft versus tumour (GvT).
19. The CD4⁺ T cell for use according to item 17 or 18 wherein said cell is modified to produce high levels of IL-10.
20. The CD4⁺ T cell for use according to items 17-19 wherein said cell is genetically modified to produce IL-10.
21. A composition comprising a CD4⁺ T cell as defined in any one of items 1 to 20 and proper excipients for use in the treatment and/or prevention of a tumor wherein said tumor expresses CD13, HLA-class I and CD54 or for use in the treatment and/or prevention of leukemia relapse or for use in a method to induce Graft versus tumour (GvT).
22. The composition according to item 21 further comprising a therapeutic agent.
23. A method to select a subject to be treated with a CD4⁺ T cell that produces high level of IL-10 comprising detecting the presence of a cell that expresses CD13, HLA-class I and CD54 in a biological sample obtained from the subject, wherein if the presence of the cell that expresses CD13, HLA-class I and CD54 is detected, the subject is selected to be treated with said CD4⁺ T cell.
24. The method according to item 23 wherein the cell further expresses CD112 and/or CD58.
25. The method according to item 23 or 24 wherein the cell further expresses CD155.
26. A kit for use in the method according to items 23 to 25 comprising means to detect the presence of a cell that expresses at least CD13, HLA-class I and CD54 in a biological sample.

The present invention will be illustrated by means of non-limiting examples referring to the following figures.

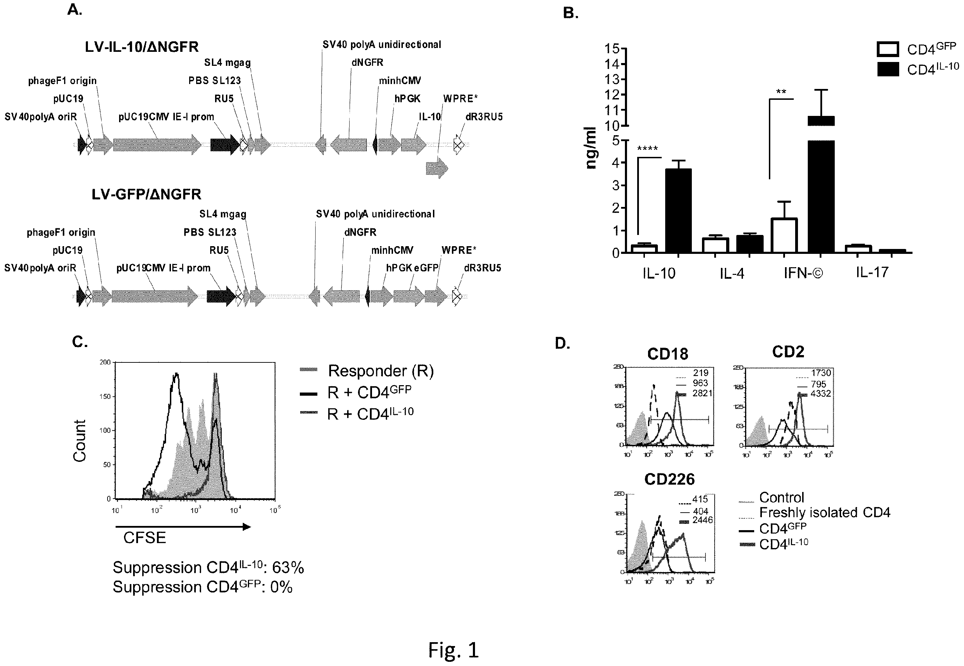

**Figure 1****. CD4^{IL-10} cells are phenotypically and functionally super-imposable to Tr1 cells. A.** Scheme of the LV-IL-10/ΔNGFR and LV-GFP/ΔNGFR vectors. The presence of the bidirectional promoter (mCMV/PGK) allows the co-regulated expression of ΔNGFR and human IL-10 or GFP genes. ψ, encapsidation signal including the 5' portion of GAG gene (GA); RRE, Rev-responsive element; cPPT, central poly-purine tract; polyA, poly-adenylation site from the Simian Virus 40; CTE, constitutive transport element; WPRE, woodchuck hepatitis virus post-transcription regulatory element. **B.** CD4^{IL-10} and CD4^{GFP} cells were stimulated with immobilized anti-CD3 and soluble anti-CD28 mAbs for 48 hours and IL-10, IL-4, IFN-γ, and IL-17 levels were measured by ELISA in culture supernatants. All samples were tested in duplicate-triplicate. Mean±SEM of n=35 for IL-10 and IL-4, n=29 for IFN-γ, and n=7 for IL-17 of CD4^{IL-10} cells and of n=19 for IL-10 and IL-4, n=16 for IFN- γ, and n=12 for IL-17 of CD4^{GFP} cells are presented. ^{∗∗} *P*≤0.01; ^{∗∗∗∗} *P*≤0.0001, Mann Whitney t-test. **C.** CD4^{IL-10} cells suppress T cell proliferation *in vitro.* Allogeneic PBMC cells were labeled with CSFE and stimulated with immobilized anti-CD3 and anti-CD28 mAbs alone (filled light grey histogram) or in the presence of CD4^{IL-10} cells (red solid line) or CD4^{GFP} cells (black solid line) at 1:1 ratio. After 4 days of culture, the percentage of proliferating PBMC was determined by CSFE dilution. One representative donor out of six tested is shown. The suppression mediated by CD4^{IL-10} cells or CD4^{GFP} cells was calculated as follows: ([proliferation responder-proliferation transduced)/proliferation responder] × 100). **D.** The expression of CD18, CD2 and CD226 on freshly isolated CD4⁺ T, CD4^{IL-10} , and CD4^{GFP} cells was evaluated by FACS. Un-stained CD4^{IL-10} cells (filled light gray histogram, isotype control), freshly isolated CD4⁺ T cells (dashed black line histogram), CD4^{GFP} cells (black line histogram), CD4^{IL-10} cells (red solid line histogram). Numbers indicate the MFI on CD4⁺ gated cells. One representative donor out of 5 tested is shown.

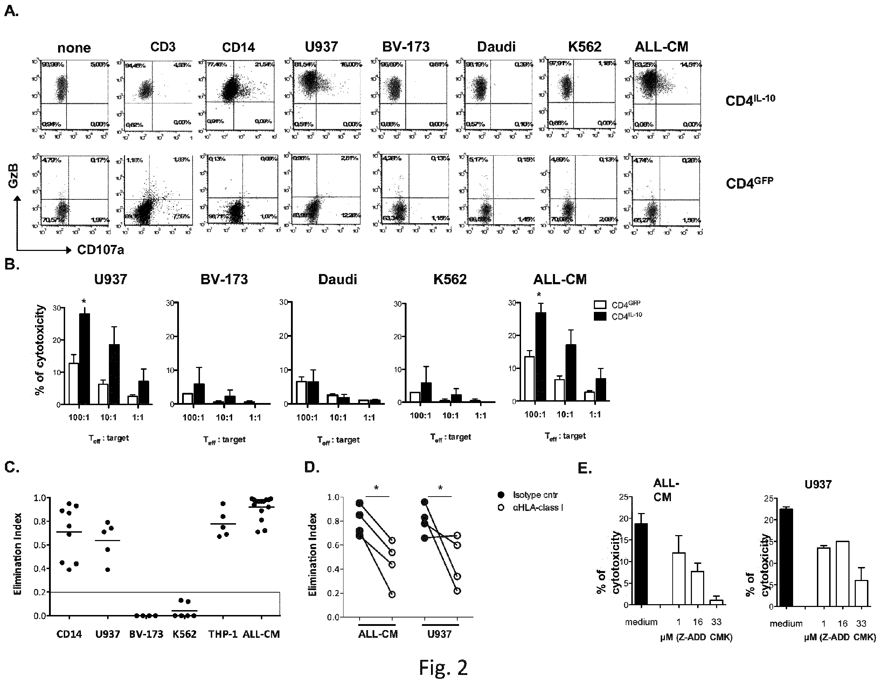

**Figure 2****. CD4^{IL-10} cells specifically lyse myeloid cell lines in HLA-class and I-GzB-dependent manner. A.** CD4^{IL-10} and CD4^{GFP} cells were co-cultured with CD3, CD14, U937, BV-173, Daudi, K562 and ALL-CM target cell lines at 5:1 (E:T) ratio. After 6 hours, degranulation of CD4^{IL-10} and CD4^{GFP} cells was measured by the co-expression of CD107a and Granzyme (GzB). Numbers in quadrants indicate percentage of positive cells, one representative donor is depicted. **B.** Cytotoxicity of CD4^{IL-10} and CD4^{GFP} cells against U937, BV-173, Daudi, K562, and ALL-CM cells was determined by ⁵¹Cr-release assay. Mean±SEM of cytotoxicity performed in duplicated is reported; mean±SEM of n=6 and of n=4 independent donors for CD4^{IL-10} and CD4^{GFP} cells against ALL-CM and U937 cells, and of n=4 and of n=2 for CD4^{IL-10} and CD4^{GFP} cells against BV-173, Daudi, and K562 cells. ^{∗}*P*<0.05, Mann Whitney t-test. **C.** CD4^{IL-10} and CD4^{GFP} cells were co-cultured with CD14, U937, BV-173, K562, THP-1, and ALL-CM cells at 1:1 ratio. After 3 days, residual leukemic cell lines (CD45^{low}CD3⁻) were analyzed and counted by FACS. Cytolysis mediated by CD4^{IL-10} cells was measured as elimination index (see Material and Methods) for each target cells. Analysis was performed at least in three independent experiments. Dots represent the elimination index of CD4^{IL-10} cells generated from different healthy donors. Lines represent mean values of the elimination index. Rectangular box indicates the threshold of cytotoxicity. **D.** CD4^{IL-10} and CD4^{GFP} cells were co-cultured with ALL-CM or U937 target cell line at 1:1 (E:T) ratio in the presence of 10 µg/ml of anti-HLA class I or isotype control mAbs. After 3 days, residual leukemic cell lines (CD45^{low}CD3⁻) were analyzed and counted by FACS. Cytolytic effect by CD4^{IL-10} cells was measured as elimination index for each target cells. Dots represent CD4^{IL-10} cells generated from different healthy donors. Lines represent mean values of the elimination index. ^{∗}*P*<0.05, Mann Whitney t-test. **E.** CD4^{IL-10} cells were preincubated with Z-AAD-CMK at the indicated concentrations and co-cultured with ALL-CM and U937 target cell at 50:1 (E:T) ratio, and cytotoxicity was determined by ⁵¹Cr release. Mean±SEM of n=3 independent donors performed in triplicates is reported.

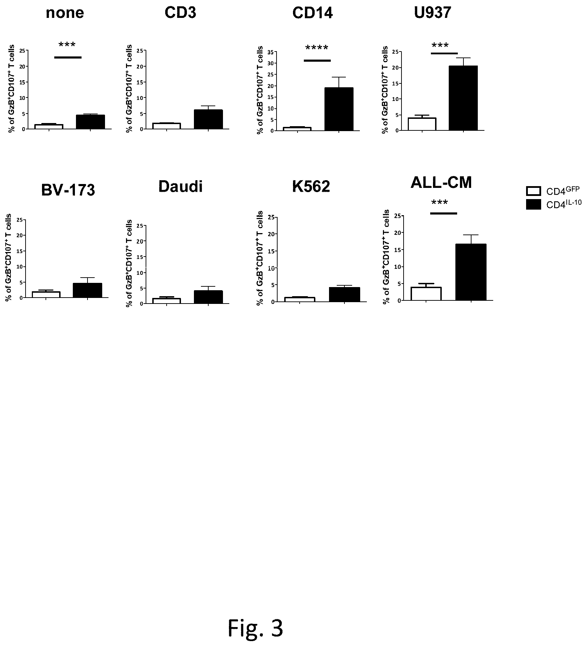

**Figure 3****. CD4^{IL-10} cells specifically degranulate when co-cultures with myeloid cells.** CD4^{IL-10} and CD4^{GFP} cells were co-cultured with CD3, CD14, U937, BV-173, Daudi, K562 and ALL-CM target cell lines at 5:1 (E:T) ratio. After 6 hours, degranulation of CD4^{IL-10} and CD4^{GFP} cells was measured by the co-expression of CD107a and Granzyme (GzB). Mean±SEM of n= 15 for CD4^{IL-10} cells, and n=12 for CD4^{GFP} cells cultured alone or with ALL-CM or U937 cells, n= 5 for CD4^{IL-10} cells and n=2 for CD4^{GFP} cells co-cultured with CD3 cells, n=10 for CD4^{IL-10} cells and n=8 for CD4^{GFP} cells co-cultured with CD14 cells, n=5 for CD4^{IL- 10} cells and n=6 for CD4^{GFP} cells co-cultured with BV-173 cells, n=4 for CD4^{IL-10} cells and n=6 for CD4^{GFP} cellsco-cultured with Daudi cells, and n=11 or CD4^{IL-10} cells and n= 9 for CD4^{GFP} cells co-cultured with K562 cells is reported. ^{∗∗∗} *P*≤0.001; ^{∗∗∗∗} *P*≤0.0001, Mann Whitney t-test.

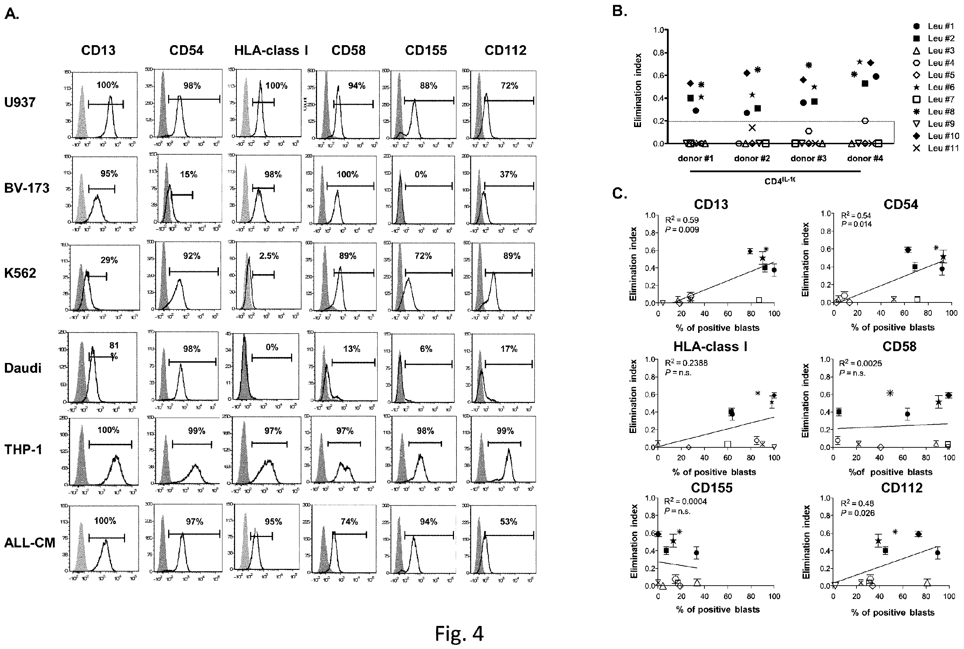

**Figure 4****. CD4^{IL-10} cells specifically kill** *in vitro* **primary blasts expressing CD13, CD54, and CD112. A.** The expression of the CD13, CD54, HLA-class I, CD58, CD155, and CD112 on freshly isolated CD14 cells, U937, BV-173, K562, Daudi, THP-1, and ALL-CM cell lines was analyzed by FACS. Numbers indicate the percentages of positive cells (black solid line) according to isotype control (filled light gray histogram). **B.** CD4^{IL-10} and CD4^{GFP} cells were co-cultured with primary blast at 1:1 (E:T) ratio. After 3 days, residual leukemic blasts (CD45^{low}CD3⁻) were analyzed and counted by FACS. Cytolytic effect of CD4^{IL-10} cells was measured as elimination index (see Material and Methods) for each target cells. Dots represent each primary blast co-cultured with CD4^{IL-10} cells. Rectangular box indicates the threshold of cytotoxicity. **C.** Correlation between cytotoxicity and expression of specific markers on primary blasts. Plots represent percentages of CD13, CD54, HLA-class I, CD58, CD155, and CD112 positive primary blasts versus elimination index of CD4^{IL-10} cells for each primary blast tested in a co-culture assay (mean±SEM). The line represents the linear regression. The P value of the correlation and the coefficient of determination (R2) are reported (two-tailed test).

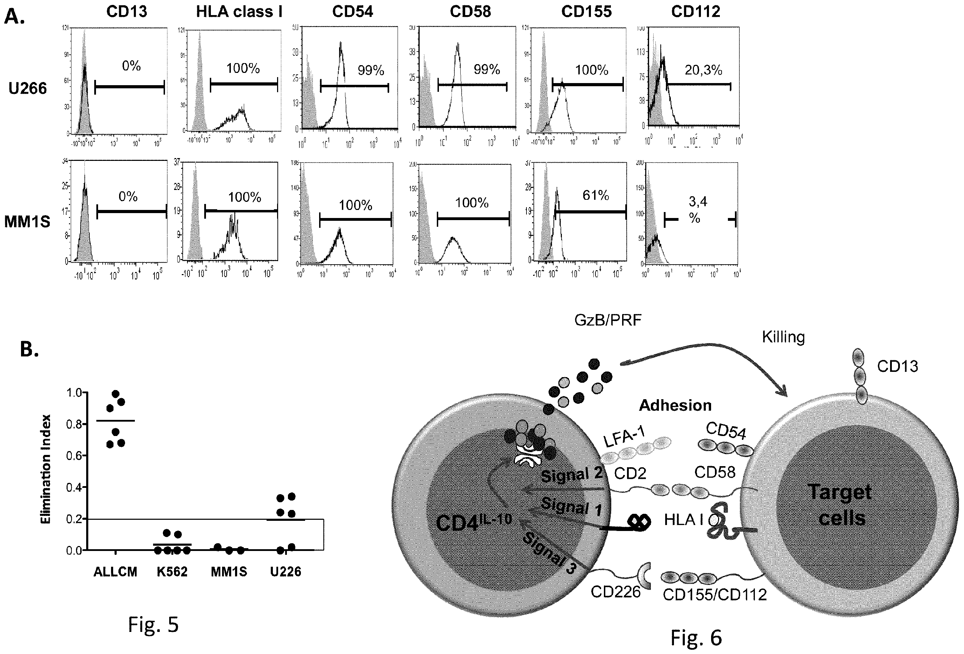

**Figure 5****. CD13 expression defines the killing specificity of CD4^{IL-10} cells. A.** The expression of the CD13, CD54, HLA-class I, CD58, CD155, and CD112 on U266, and MM1S cell lines was analyzed by FACS. Numbers indicate the percentages of positive cells (black solid line) according to isotype control (filled light gray histogram). **B.** CD4^{IL-10} and CD4^{GFP} cells were co-cultured with ALL-CM, K562, MM1S, and U266 cells at 1:1 ratio. After 3 days, residual leukemic cell lines (CD45^{low}CD3⁻) were analyzed and counted by FACS. Cytolysis mediated by CD4^{IL-10} cells was measured as elimination index (see Material and Methods) for each target cells. Analysis was performed at least in three independent experiments. Dots represent the elimination index of CD4^{IL-10} cells generated from different healthy donors. Lines represent mean values of the elimination index. Rectangular box indicates the threshold of cytotoxicity.
**Figure 6****. The molecular mechanism underlying the lytic activity of CD4^{IL-10} cells is comparable to that of *bona fide* Tr1 cells.** This mechanism requires stable adhesion between CD4^{IL-10} cells and CD13⁺ blasts mediated by LFA-1/CD54 interaction, activation of CD4^{IL-10} cells *via* HLA class I (Signal 1), *via* CD2 (Signal 2), and *via* CD226 (Signal 3), leading to GzB release and killing of leukemic blasts.

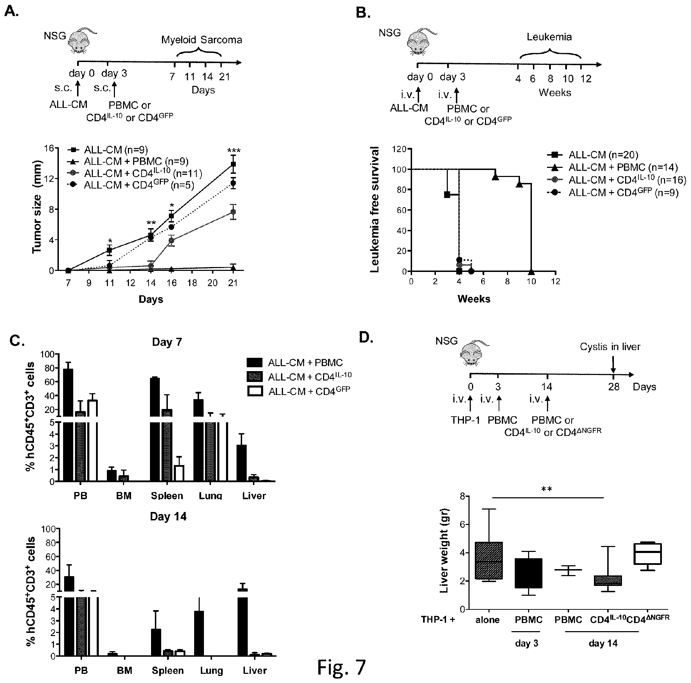

**Figure 7****. The *in vivo* localization of CD4^{IL-10} cells influences their anti-leukemic activity. A.** CD4^{IL-10} cells delayed the subcutaneous ALL-CM tumor growth. NSG mice were sub-cutaneously injected with ALL-CM (2×10⁶cells/mouse). Three days later mice received allogeneic PBMC (2×10⁶cells/mouse), or CD4^{IL-10} cells, or CD4^{GFP} cells (1×10⁶cells/mouse). Tumor growth was measured at the indicated time points after ALL-CM injection. Statistical analysis were perform by comparing CD4^{IL-10} cells treated mice (ALL-CM + CD4^{IL-10}) versus un-treated control mice (ALL-CM): ^{∗}*P*<0.05, ^{∗∗}*P*≤ 0.01; ^{∗∗∗} *P*≤0.001; two-way ANOVA plus Bonferroni post test. **B.** CD4^{IL-10} cells do not mediate GvL effect in the ALL-CM leukemia model of T-cell therapy. NSG mice were intravenously injected with ALL-CM (5×10⁶ cells/mouse) alone or, on day three, with allogeneic PBMC (5×10⁶cells/mouse), CD4^{IL-10} cells, or CD4^{GFP} cells (2.5×10⁶cells/mouse). Leukemia free survival (presence of more than 50% of circulating human blast hCD45⁺hCD3⁻ in peripheral blood) was followed over time after cell injection. Data obtained from four independent experiments are presented. **C.** *In vivo* localization of CD4^{IL-10} cells. NSG mice were intravenously injected with ALL-CM (5×10⁶ cells/mouse) alone or, on day three, with allogeneic PBMC (5×10⁶cells/mouse), CD4^{IL-10} cells, or CD4^{GFP} cells (2.5×10⁶cells/mouse). On day 7 and 14 the percentages of human T cells (hCD45⁺hCD3⁺) in peripheral blood, bone marrow, spleen, lung, and liver were evaluated by FACS. Mean±SD of n=6 mice for ALL-CM + PBMC and for ALL-CM + CD4^{GFP} cells, and n=7 mice for ALL-CM + CD4^{IL-10} cells in the peripheral blood on day 7, and mean±SD n=3 mice for ALL-CM + PBMC and ALL-CM + CD4^{GFP} cells, and n=4 mice for ALL-CM + CD4^{IL-10} cells, on day 7 in other organs (upper panel). Mean±SD of n=3 mice per group on day 14 (lower panel). D. CD4^{IL-10} cells mediate anti-leukemic effect in a model of extramedullary tumors. NSG mice were intravenously injected with THP-1 leukemia cells (2×10⁶cells/mouse). Three days later mice received allogeneic PBMC (2×10⁶cells/mouse), or fourteen days later mice were injected with CD4^{IL-10} cells, or CD4^{GFP} cells (1×10⁶cells/mouse). Tumor growth was analyzed by measuring the weights of THP-1-infiltrated livers. Mean±SEM of n=6 mice for THP-1, n=5 mice THP-1 + PBMC, n=4 mice for THP-1 + CD4^{IL-10} cells, and n=2 mice for THP-1 + CD4^{GFP} cells are presented.

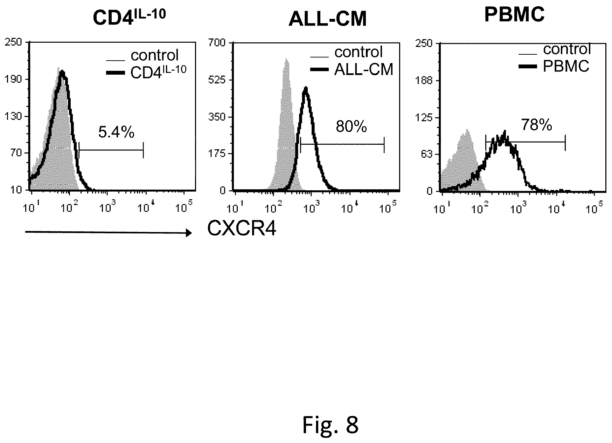

**Figure 8****. CD4^{IL-10} cells do not express CXCR4.** CD4^{IL-10} cells, ALL-CM, and PBMC were analyzed for the expression of CXCR4 and of CD62L by FACS. Numbers indicate the percentage of positive cells (black solid line) according to isotype control (filled light gray histogram). One representative out of three tested is shown.

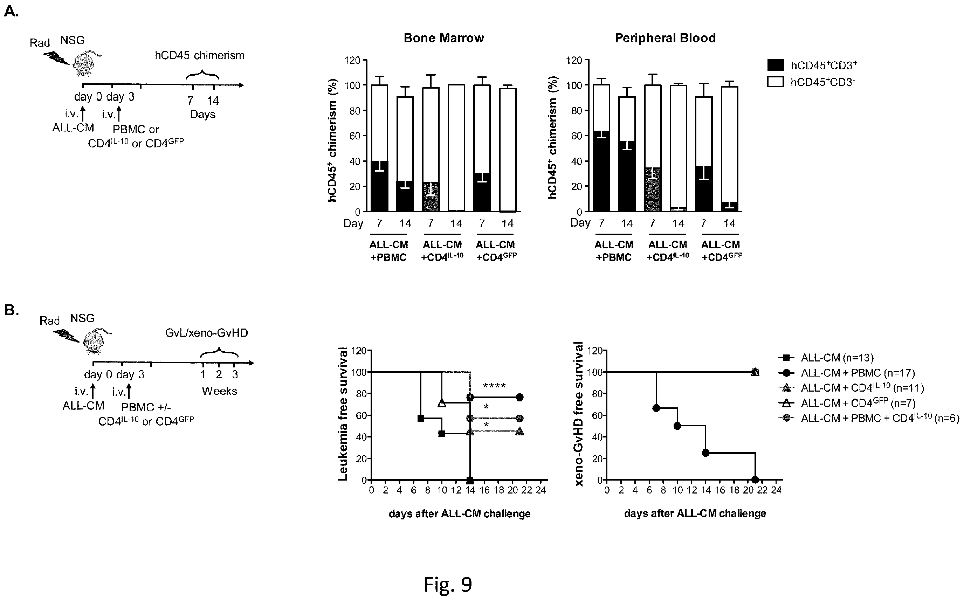

**Figure 9****. Adoptive transfer of CD4^{IL-10} cells prevents GvHD while spearing the Graft-versus-Leukemia of allogeneic T cells. A.** *In vivo* localization of CD4^{IL-10} cells in conditioned mice. NSG mice were sub-lethally irradiated and intravenously injected with ALL-CM (5×10⁶cells/mouse). Three days later mice received allogeneic PBMC (5×10⁶cells/mouse), or CD4^{IL-10} cells, or CD4^{GFP} cells (2.5×10⁶cells/mouse). On day 7 and 14 the percentages of human T cells (hCD45⁺hCD3⁺) and ALL-CM (hCD45⁺hCD3⁻) in bone marrow and peripheral blood were evaluated by FACS. Mean±SEM of n=9 mice for ALL-CM + PBMC, and for ALL-CM + CD4^{IL-10} cells, and n=7 for ALL-CM + CD4^{GFP} cells on day 7, and n= 14 for ALL-CM + PBMC, n= 11 for ALL-CM + CD4^{IL-10} cells, and n=7 for ALL-CM + CD4^{GFP} cells on day 14 in the bone. Mean±SEM of n=17 mice for ALL-CM + PBMC and for ALL-CM + CD4^{IL-10} cells, and n=10 for ALL-CM + CD4^{GFP} cells on day 7, and n= 16 for ALL-CM + PBMC, n= 14 for ALL-CM + CD4^{IL-10} cells, and n=8 for ALL-CM + CD4^{GFP} cells on day 14 in the peripheral blood. Data were obtained from two to five independent experiments. **B.** Adoptive transfer of CD4^{IL-10} cells prevents xeno-GvHD while sparing GvL mediated by human allogeneic PBMC. NSG mice were sub-lethally irradiated and intravenously injected with ALL-CM (5×10⁶cells/mouse). Three days later mice received allogeneic PBMC (5×10⁶cells/mouse) alone or in combination with CD4^{IL-10} cells (2.5×10⁶cells/mouse). As control mice were injected with CD4^{IL-10} cells or CD4^{GFP} cells (2.5×10⁶cells/mouse) alone. Leukemia free survival (presence of more than 50% of circulating human blast hCD45⁺hCD3⁻ in peripheral blood) and xeno-GvHD free survival (presence of more than 50% of circulating human T lymphocytes hCD45⁺hCD3⁺ in peripheral blood with a loss of weight higher than 20%) was followed over time after cell injection. Statistical analysis were perform by comparing treated mice versus un-treated control mice (ALL-CM): ^{∗}*P*<0.05, ^{∗∗∗∗} *P*≤0.0001; two-way ANOVA plus Bonferroni post-test.

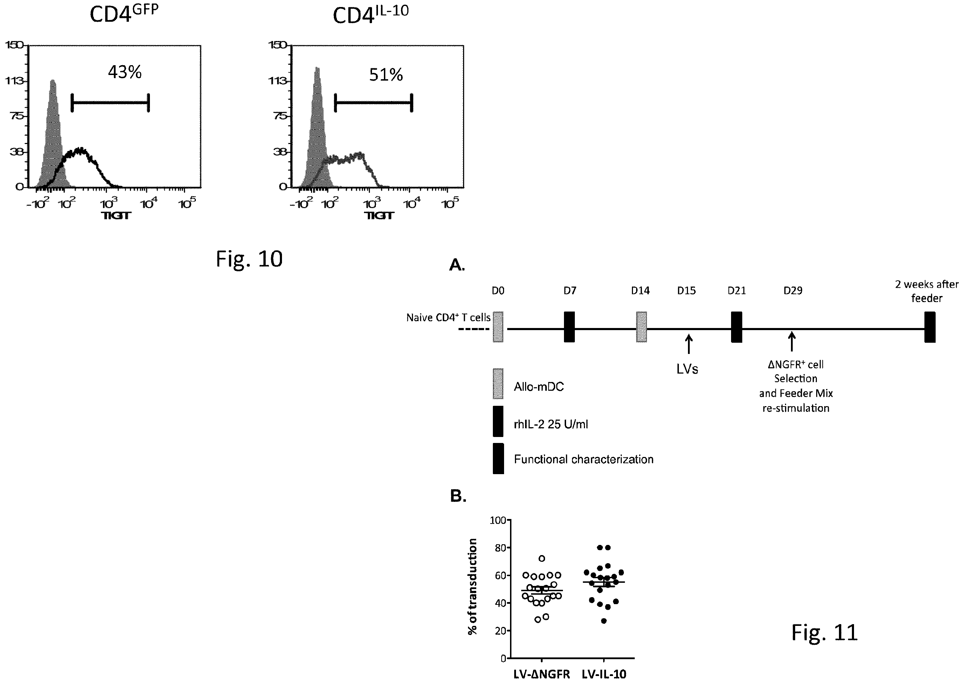

**Figure 10****. CD4^{IL-10} cells express TIGIT** (T cell immunoreceptor with Ig and ITIM domains). CD4^{GFP} and CD4^{IL-10} cells were analyzed for the expression of TIGIT by FACS. Numbers indicate the percentage of positive cells (CD4^{GFP} cells, black solid line, CD4^{IL-10} cells, red solid line) according to isotype control (filled light gray histogram). One representative out of three tested is shown.
**Figure 11****. Enriched allo-specific CD4⁺ T cells are efficiently transduced with LV-IL-10. A.** Protocol to generate and characterize allo-CD4^{IL-10} cells. Enriched allo-specific CD4⁺ T cells were transduced with LV-IL-10 or LV-GFP during a secondary stimulation with the same allo-mDC used for priming (see also Material and Methods). Human CD4⁺ T cells were stimulated with allo-mDC for 14 days. After culture, CD4⁺ T cells were re-stimulated with allo-mDC 24 hours before transduction with LV-IL-10 or LV-GFP at MOI of 20. **B.** Analysis of the transduced cells analyzed by FACS at day 6 after the transduction. The frequencies of ΔNGFR⁺ cells are indicated. Collective results from individual donors are presented. Each dot represent a single donor tested, lines indicate the mean±SEM of the percentage of transduction.

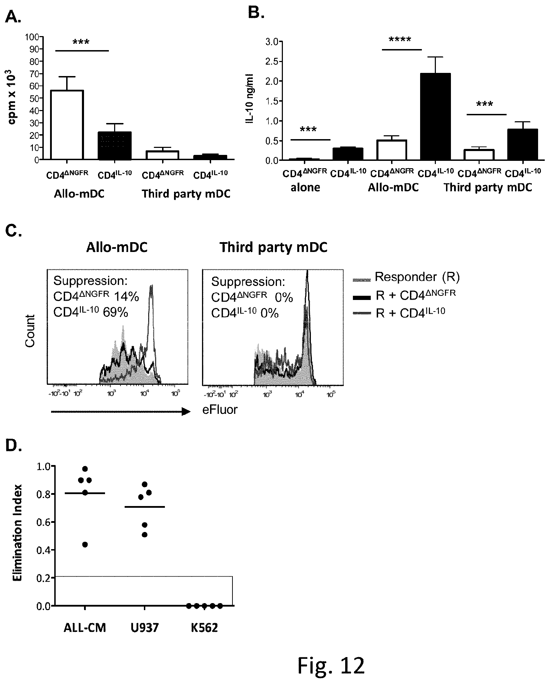

**Figure 12****. Enforced IL-10 expression in allo-specific CD4⁺ T cells promotes their conversion into Tr1-like cells with the ability to kill myeloid cells. A.** The proliferative responses were evaluated 3 or 5 days after culture of allo-CD4^{IL-10} and allo-CD4^{ΔNGFR} cells with allogeneic (allo-mDC) or third party mDC, respectively, by ³HThy incorporation for additional 16 hours. Bars represent mean value with SEM of n=19 for allogeneic stimulation and n=16 for third party stimulation tested in more than 5 independent experiments. All samples were tested in duplicate-triplicate, ^{∗∗∗} *p*<0.001, Wilcoxon matched-pairs signed rank test. **B.** Allo-CD4^{IL-10} or allo-CD4^{ΔNGFR} cells were left inactivated or stimulated with the same allo-mDC used for priming or a third party mDC (ratio 10:1), and IL-10 production was determined by ELISA 48 hours after activation. Bars represent the mean value with SEM of n= 12-26 donors tested in 5 independent experiments. All samples were tested in duplicate-triplicate, ^{∗∗∗} *p*<0.001, ^{∗∗∗∗} *p*<0.0001 Wilcoxon matched-pairs signed rank test. **C.** Allo-CD4^{IL-10} or allo-CD4^{ΔNGFR} cells were tested for their ability to suppress proliferation of autologous CD4⁺ T cells primed with allo-mDC. Autologous primed CD4⁺ T cells (Responders) were labeled with eFluor dye and stimulated with allo-mDC (ratio 10:1) alone or in the presence of allo-CD4^{IL-10} or allo-CD4^{ΔNGFR} cells at 1:1 ratio. After 3 days of culture the suppressive ability was determined by analyzing the eFluor dye dilution. One representative donor out of four tested is shown in the left panel. The suppression mediated by allo-CD4^{IL-10} cells or allo-CD4^{ΔNGFR} cells was calculated as follows: ([proliferation responder-proliferation transduced)/proliferation responder] × 100). On the right panel, dots represent the suppression of allo-CD4^{IL-10} cells or allo-CD4^{ΔNGFR} cells generated from different healthy donors. Lines represent mean value±SEM of % of suppression. **D.** CD4^{IL-10} and CD4^{ΔNGFR} cells were co-cultured with ALL-CM, U937, K562, mDC allo or mDC third party as target cells at 1:1 ratio. After 3 days, residual leukemic cell lines (CD45^{low}CD3⁻) were analyzed and counted by FACS. Cytolysis mediated by CD4^{IL-10} cells was measured as elimination index (see Material and Methods) for each target cells. Analysis was performed in two independent experiments. Dots represent the elimination index of CD4^{IL-10} cells generated from different healthy donors co-cultured with n=5 ALL-CM cells, n=6 U937 cells, n=6 K562 cells, n=4 mDC allo, and n=4 mDC third party. Lines represent mean values of the elimination index. Rectangular box indicates the threshold of cytotoxicity.

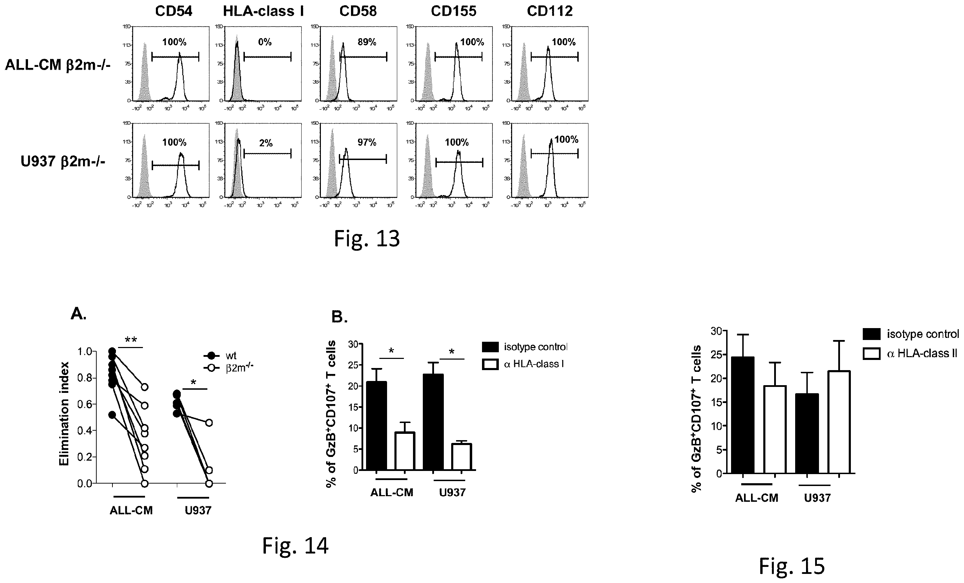

**Figure 13****. HLA-class I expression is abolished by β2 microglobulin gene disruption.** The expression of the CD54, HLA-class I, CD58, CD155, and CD112 on β2m^{-/-} ALL-CM and β2m^{-/-} U937 cell lines was analyzed by FACS. Numbers indicate the percentages of positive cells (black solid line) according to isotype control (filled light gray histogram). Data representative of at least three independent experiments are shown.
**Figure 14****. CD4^{IL-10} cell-mediated killing of myeloid cell lines is HLA-class I-dependent. A.** CD4^{IL-10} and CD4^{ΔNGFR} cells were co-cultured with ALL-CM, β2m^{-/-} ALL-CM, U937 or β2m^{-/-} U937 target cell line at 1:1 (E:T) ratio. After 3 days, residual leukemic cell lines (CD45^{low}CD3⁻) were analyzed and counted by FACS. Cytolytic effect by CD4^{IL-10} cells was measured as elimination index for each target cells. Dots represent CD4^{IL-10} generated from n=8 different healthy donors co-cultured with ALL-CM and β2m^{-/-} ALL-CM, n=5 different healthy donors co-cultured with U937 and β2m^{-/-} U937 tested in two independent experiments. ^{∗} *P*<0.05, ^{∗∗} *P*<0.001 two-sided Wilcoxon matched-pairs signed rank test. **B.** CD4^{IL-10} and CD4^{ΔNGFR} cells were co-cultured with ALL-CM or U937 target cell line at 5:1 (E:T) ratio in the presence of 10 µg/ml of anti-HLA class I or isotype control mAbs. After 6 hours, degranulation of CD4^{IL-10} and CD4^{ΔNGFR} cells was measured by the co-expression of CD107a and Granzyme (GzB). A Mean±SEM of GzB⁺CD107a⁺ CD4^{IL-10} cell, generated from eight different healthy donors tested in two independent experiments, is reported. ^{∗} *P*<0.05, two-sided Wilcoxon matched-pairs signed rank test.
**Figure 15****. CD4^{IL-10} cell-mediated killing of myeloid cell lines is TCR-independent .** CD4^{IL-10} and CD4^{ΔNGFR} cells were co-cultured with ALL-CM or U937 target cell line at 5:1 (E:T) ratio in the presence of 10 µg/ml of anti-HLA class II or isotype control mAbs. After 6 hours, degranulation of CD4^{IL-10} and CD4^{ΔNGFR} cells was measured by the co-expression of CD107a and Granzyme (GzB). A Mean±SEM of GzB⁺CD107a⁺ CD4^{IL-10} cell, generated from four different healthy donors tested in two independent experiments, is reported.

### Detailed description of the invention

### Material and Methods

**Plasmid construction.** The coding sequence of human IL-10 was excised from pH15C (ATCC n° 68192). The resulting 549bp fragment was cloned into the multiple cloning site of pBluKSM (Invitrogen) to obtain pBluKSM-hIL-10. A fragment of 555bp was obtained by excision of hIL-10 from pBluKSM-hIL-10 and ligation to 1074.1071.hPGK.GFP.WPRE.mhCMV.dNGFR.SV40PA (here named LV-ΔNGFR), to obtain LV-IL-10/ΔNGFR. The presence of the bidirectional promoter (human PGK promoter plus minimal core element of the CMV promoter in opposite direction) allows co-expression of the two transgenes. The sequence of LV-IL-10/ ΔNGFR was verified by pyrosequencing (Primm).

**Vector production and titration.** VSV-G-pseudotyped third generation LVs were produced by Ca₃PO₄ transient four-plasmid co-transfection into 293T cells and concentrated by ultracentrifugation as described ¹⁹ with a small modification: 1 µM sodium butyrate was added to the cultures for vector collection. Titer was estimated on 293T cells by limiting dilution, and vector particles were measured by HIV-1 Gag p24 antigen immune capture (NEN Life Science Products; Waltham, MA). Vector infectivity was calculated as the ratio between titer and particle. For concentrated vectors, titers ranged from 5×10⁸ to 6×10⁹ transducing units/ml, and infectivity from 5×10⁴ to 10⁵ transducing units/ng of p24.

**Patients and donors.** All protocols were approved by the Institutional Review Board and samples collected under written informed consent according to the Declaration of Helsinki. Patient characteristics are listed in Table 1.

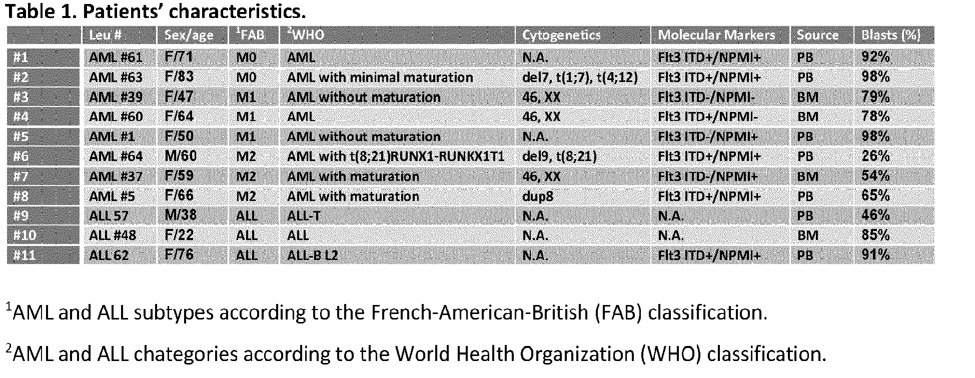

N.A. Not Applicable; Flt3 ITD, Flt3 internal-tandem duplication; NPM1, nucleophosmin; BM, bone marrow, PB, peripheral b

Peripheral blood mononuclear cells (PBMC) were prepared by centrifugation over Ficoll-Hypaque gradients. CD4⁺ T cells were purified by negative selection with the CD4 T cell isolation kit (Miltenyi Biotec, Bergisch Gladbach, Germany) with a resulting purity of >95%. CD14⁺ and CD3⁺ T cells were purified by positive selection with CD14⁺ and CD3⁺ Microbeads (Miltenyi Biotec, Bergisch Gladbach, Germany) with a resulting purity of >95%. U937 (monocytic cell line), K562 (erythroleukemic cell line), BV-173 (a pre-B lymphoblastic leukemia ²⁰, Daudi (B lymphoblastic cell line), THP1 (myelomonocytic leukemia) cell lines were obtained from the ATCC. ALL-CM cell line derived from a CML patient suffering from a Philadelphia chromosome-positive lymphoid blast crisis is described in Bondanza et al.⁴¹. To generate 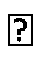 2m-deficient ALL-CM and U937 cell lines, cells were nucleofected by Amaxa 4D Nucleofector System with X-unit (LONZA group ltd, CH) using EP100 program. Briefly, 3×10⁵ cells were re-suspended in a solution containing 20 µl of SF solution (LONZA) and 3 µl of pre-mixed Cas9 plasmid (500 ng) and the specific B2M guide #18 CRISPR plasmid (GAGTAGCGCGAGCACAGCTA (SEQ ID No. 1) cut in B2M exon1, 250 ng). After nucleofection, cells were expanded in culture. All cell lines were routinely tested for mycoplasma contamination.

**Transduction of human CD4⁺ T cells.** CD4⁺ purified T cells were activated for 48 hours with soluble anti-CD3 monoclonal antibody (mAb, 30 ng/ml, OKT3, Miltenyi Biotec, Bergisch Gladbach, Germany), anti-CD28 mAb (1 µg/ml, BD, Biosciences) and rhlL-2 (50 U/ml, Chiron, Italy) and transduced with LV-GFP/ΔNGFR (CD4^{GFP}), LV-IL-10/ΔNGFR (CD4^{IL-10}) with MOI of 20 as previously described ¹⁸. Transduced CD4⁺ΔNGFR⁺ T cells were purified 14 days after transduction by FACS-sorting or using CD271⁺ Microbeads (Miltenyi Biotec, Bergisch Gladbach, Germany) and expanded in X-VIVO 15 medium supplemented with 5% human serum (BioWhittaker-Lonza, Washington, DC), 100 U/ml penicillin-streptomycin (BioWhittaker), and 50 U/ml rhIL-2. CD4^{GFP} and CD4^{IL-10} cells were stimulated every two weeks in the presence of an allogeneic feeder mixture containing 10⁶ PBMC (irradiated at 6,000 rad) per ml, 10⁵ JY cells (an Epstein-Barr virus-transformed lymphoblastoid cell line expressing high levels of human leukocyte antigen and co-stimulatory molecules, irradiated at 10,000 rad) per ml, and soluble anti-CD3 mAb (1 µg/ml). Cultures were maintained in 50-100 U/ml rhlL-2 (PROLEUKIN, Novartis, Italy). All FACS phenotypic analysis, *in vitro* and *in vivo* experiments were performed in cells from at least 12 days after feeder addition, in resting state.

To generate alloantigen-specific transduced cells, naive CD4⁺ T cells (10⁶/well) were stimulated with allogeneic mature dendritic cells (mDC) (10⁵/well) in a final volume of 1 mL in 24-well plates. At day 7 and 10, half of the medium was replaced by fresh medium supplemented with 25 U/ml of rhIL-2, and at day 14, cells were collected, washed and transduced with LV-GFP/ΔNGFR (CD4^{ΔNGFR}) or LV-IL-10/ΔNGFR (CD4^{IL-10}) with MOI of 20 after 24 hours of secondary stimulation with the same allo-mDC used for priming. Transduced CD4⁺ΔNGFR⁺ T cells were purified and expanded as above.

**Cytokine determination.** To measure cytokine production, CD4^{GFP} and CD4^{IL-10} cells were stimulated with immobilized anti-CD3 (10 µg/ml) and soluble anti-CD28 (1 µg/ml) mAbs in a final volume of 200 µl of medium (96 well round-bottom plates, 2×10⁵/well). Culture supernatants were harvested after 48 hours of culture and levels of IL-4, IL-10, IFN-γ and IL-17, were determined by ELISA according to the manufacturer's instructions (BD Biosciences).

**Suppression assays.** To test the suppressive capacity of CD4^{GFP} and CD4^{IL-10} cells, allogeneic PBMC were labeled with CFSE (Molecular Probes) or eFluor670 (Invitrogen) before stimulation with immobilized anti-CD3 (10 µg/ml) and soluble anti-CD28 (1 µg/ml) mAbs. Suppressor cells were added at 1:1 ratio. After 4-6 days of culture, proliferation of CFSE/eFluor670-labeled responder cells was determined by flow cytometry.

**Cytotoxicity assays.** T-cell degranulation was evaluated in a CD107a flow cytometric assay, according to the protocol described in ⁶. In some experiments anti-HLA-class I (clone W6/32, Biolegend, USA) and isotype control (IgG2a,k, BD Pharmigen, USA) mAbs were added at the indicated concentrations. The cytotoxic activity of CD4^{GFP} and CD4^{IL-10} cells was analyzed in a standard ⁵¹Cr-release assay as described in detail elsewhere. Briefly, 10^{3 51}Cr-labeled (NEN Dupont, Milan, Italy) target ALL-CM, BV-173, Daudi, U937 or K562 cells were incubated for 4 hours with CD4^{GFP} and CD4^{IL-10} cells at various effector-target cell ratios, plated in duplicate. Subsequently, the supernatant was removed and counted on a γ counter. Percentage of specific lysis was calculated according to the formula: 100×(⁵¹Cr experimental release - spontaneous release)/(maximum release - spontaneous release). In some experiments Z-AAD-CMK (Sigma, CA, USA) was added at the indicated concentrations. Alternatively, cytotoxicity of CD4^{GFP} and CD4^{IL-10} cells was analysed in co-culture experiments. Briefly, target and effector cells (CD4^{GFP} and CD4^{IL-10} cells) were plated in a ratio 1:1 for 3 days. At the end of co-culture, cells were harvested and surviving cells were counted and analysed by FACS. Elimination index (EI) was calculated as 1-(number of target remained in the co-culture with CD4^{IL-10}/number of target remained in the co-culture with CD4^{GFP}). In some experiments anti-HLA-class I (clone W6/32, Biolegend, USA) and isotype control (IgG2a,k, BD Pharmigen, USA) mAb were added at the indicated concentrations.

**Flow cytometry analysis.** For the detection of cell surface antigens CD4^{IL-10} and CD4^{GFP} cells were stained with anti-CD4 (BD Pharmingen, USA), anti-CD226 (Biolegend, USA), anti-CD2, anti-CD18, anti-CXCR4 (BD Pharmingen, USA) mAbs after a 2.4G2 blocking step. For the detection of cell surface antigens on target cells, leukemic cell lines and primary blasts were stained with anti-CD45, anti-HLA-class I, anti-CD112, anti-CD155 (Biolegend, USA), anti-CD13, anti-CD54, anti-CD58 (BD Pharmigen, USA). Cells were incubated with the aforementioned mAbs for 30 min at 4°C in PBS 2% FBS, washed twice and fixed with 0.25% formaldehyde. To evaluate human chimerism in peripheral blood of treated NSG mice, cells were co-stained with anti-human CD45 (Biolegend), anti-human CD3 (BD Bioscience), anti-human CD33 (Miltenyi Biotech), anti-CD271 (Miltenyi Biotech) and anti-murine CD45 (BD Bioscience) mAbs.

Samples were acquired using a FACS Canto II flow cytometer (Becton Dickinson, USA), and data were analyzed with FCS express (De Novo Software, CA, USA). The inventors set quadrant markers to unstained controls.

**Graft-versus-Leukemia model, ALL-CM leukemia model:** 6- to 8-week-old female NSG mice were obtained from Charles-River Italia (Calco, Italy). The experimental protocol was approved by the internal committee for animal studies of the inventors institution (Institutional Animal Care and Use Committee [IACUC #488]). On day 0 mice were infused with ALL-CM leukemia (5×10⁶) and three days after with either allogeneic PBMC (5×10⁶) or CD4^{IL-10} or CD4^{GFP} cells (2.5×10⁶). Cells were re-suspended in 250 µl of PBS and infused intravenously. Survival and weight loss was monitored at least 3 times per week as previously described ²⁰ and moribund mice were humanely killed for ethical reasons. At weekly intervals, mice were bled and human chimerism was determined by calculating the frequency on human CD45⁺ cells within the total lymphocyte population. In some experiments mice were euthanized at day 7 and 14 after ALL-CM injection to analyse human cells distribution in different organs.

**Graft-versus-Leukemia model, THP-1 leukemia model:** 6- to 8-week-old female NSG mice were obtained from Charles-River Italia (Calco, Italy). The experimental protocol was approved by the internal committee for animal studies of the inventors institution (Institutional Animal Care and Use Committee [IACUC #488]). On day 0 mice were infused with THP-1 leukemia (2×10⁶) and three days after with allogeneic PBMC (2×10⁶) or fourteen days after with CD4^{IL-10} or CD4^{GFP} cells (1×10⁶). Cells were re-suspended in 250 µl of PBS and infused intravenously. Survival and weight loss was monitored at least 3 times per week as previously described ²⁰ and moribund mice were humanely killed for ethical reasons. Mice were euthanized at week 5 after THP1 injection to analyse human cells in the liver.

**Subcutaneous ALL-CM tumor model:** 6- to 8-week-old female NSG mice were used. The experimental protocol was approved by the internal committee for animal studies of San Raffaele Scientific Institute (Institutional Animal Care and Use Committee [IACUC #488]). On day 0 mice were infused with ALL-CM (2×10⁶) cells and three days later with allogeneic PBMC (2×10⁶) or with CD4^{IL-10} cells (1×10⁶) or CD4^{GFP} cells (1×10⁶). Cells were re-suspended in 1 ml of PBS and infused sub-cutaneously. Sarcoma growth was monitored by measurements at least 3 times per week and moribund mice were humanely killed for ethical reasons.

**Graft-versus-Leukemia and Graft-versus Host Disease model:** 6- to 8-week-old female NSG mice were used. The experimental protocol was approved by the internal committee for animal studies of San Raffaele Scientific Institute (Institutional Animal Care and Use Committee [IACUC #488]). At day 0, mice received total body irradiation with a single dose of 175-200 cGy irradiation from a linear accelerator according to the weight of mice, and were immediately infused with ALL-CM (5×10⁶). On day 3 mice were then injected with allogeneic PBMC (5×10⁶) alone or in the presence of with CD^{GFP} and CD4^{IL-10} cells (2.5×10⁶). Cells were re-suspended in 250 µl of PBS and infused intravenously. Survival and weight loss was monitored at least 3 times per week as previously described ²⁰ and moribund mice were humanely killed for ethical reasons. At weekly intervals, mice were bled and human chimerism was determined by calculating the frequency on human CD45⁺ cells within the total lymphocyte population. In some experiments mice were euthanized at day 7 and 14 after ALL-CM injection to analyse human cells distribution in different organs.

**Statistical Analysis.** Statistical analyses on the functional data were performed using a Mann-Whitney U test for non-parametric data and using a two-way analysis of variance test. ANOVA tests and Bonferroni's multiple comparisons were used to analyze the data from the *in vivo* experiments. P values less than 0.05 were considered significant. Statistic calculations were performed with the Prism program 5.0 (GraphPad Software).

### Results

**CD4^{IL-10} cells kill myeloid cells in HLA-class I- and GzB-dependent manner.** The inventors generated CD4^{IL-10} cells by transducing CD4⁺ T cells with a novel bidirectional LV co-encoding *human IL-10* and *ΔNGFR,* as clinical grade marker gene **(****Figure 1A****).** As control, T cells were transduced with a bidirectional LV co-encoding for *GFP* (in IL-10 position) and *ΔNGFR* **(****Figure 1A****).** As previously described ¹⁸, enforced expression of human IL-10 into CD4⁺ T cells allows the generation of cells (CD4^{IL-10} cells) superimposable to Tr1 cells. CD4^{IL-10} cells, in contrast to control LV-transduced CD4^{GFP} cells, secreted significantly higher levels of IL-10 (p≤0.0001). Significantly higher levels of IFN-γ (p≤0.01) and comparable low amounts of IL-4 and IL-17 **(****Figure 1B****)** were observed. It is believed that the properties, characteristics and biological activities of CD4^{IL-10} cells are due to the high level of IL-10 produced. CD4^{IL-10} cells, but not CD4^{GFP} cells, suppressed T-cell responses *in vitro* **(****Figure 1C****).** CD4^{IL-10} cells expressed CD18, which in association with CD11a forms LFA-1, CD2 and CD226 at levels higher than those detected on memory freshly isolated CD4⁺ T cells and on CD4^{GFP} cells **(****Figure 1D****)**.

To evaluate the ability of CD4^{IL-10} cells to kill transformed myeloid cells, the inventors tested a panel of leukemic cell lines. Freshly isolated T lymphocytes (CD3) and monocytes (CD14) were used as negative and positive control, respectively. The inventors first evaluated the degranulation of CD4^{IL-10} and CD4^{GFP} cells by the co-expression of GzB and the lysosomal-associated membrane protein1 (LAMP-1 or CD107a), a marker of cytotoxic degranulation in NK cells and cytotoxic T lymphocytes. When CD4^{IL-10} cells were co-cultured with CD14, U937, a monocytic cell lines, and ALL-CM, a cell line derived from a patient suffering from a lymphoblastic crisis of chronic myelogenous leukemia ^{20,21}, a significantly higher proportion of GzB⁺CD107a⁺ cells was detected (a minimum of nine donors were tested, CD4^{IL-10} versus CD4^{GFP}, p≤0.001 and p≤0.0001, respectively, **Figure 2A** **and** **3****).** The frequency of GzB⁺CD107a⁺ cells generated in co-culture with U937 and ALL-CM cells was also significantly higher compared to the spontaneous degranulation of CD4^{IL-10} cells (p≤0.001 and p≤0.0001, paired T-test, respectively). Conversely, CD4^{IL-10} cells did not degranulate when co-cultured with CD3, BV-173, a pre-B lymphoblastic leukemia ²², Daudi, a B lymphoblastic cell line, or K562, an erythroleukemic cell line (Figure 2A and 3). Consistent with the activation and the release of GzB, CD4^{IL-10} cells lysed at 100:1 (T^{eff}:target) ratio U937 and ALL-CM cells at significantly higher levels as compared to CD4^{GFP} cells (p<0.05). Conversely, CD4^{IL-10} cells did not lyse BV-173, Daudi, or K562 cells **(****Figure 2B****).** Similarly, in co-culture experiments CD4^{IL-10} cells eliminated CD14, U932, ALL-CM, and THP-1, a prototypic monocytic cell line, but not BV-173 or K562 cells **(****Figure 2C****).**

Addition of a pan anti-HLA-class I (clone W6/32) mAb inhibited the degranulation of CD4^{IL-10} cells (data not shown), and significantly prevented the killing of ALL-CM and U937 cells (p<0.05) **(****Figure 2D****).** Moreover, CD4^{IL-10}-mediated killing of ALL-CM and U937 cells was GzB-dependent, since addition of the GzB inhibitor Z-AAD-CMK inhibited the lysis in a dose-dependent manner **(****Figure 2E****).** Overall, these data showed that CD4^{IL-10} cells lysed myeloid cell lines, but not pre-B lymphoblastic or erythroblastic cell lines, *via* a mechanism that requires HLA class I-mediated activation and is GzB-dependent.

**CD^{IL-10} cells specifically kill CD13⁺ leukemic blasts that express CD54 and CD112 *in vitro.*** The inventors next investigated the phenotype of leukemic cell lines. Results indicated that U937, THP-1, and ALL-CM cells target of CD4^{IL-10}-mediated lysis were CD13⁺ and expressed CD54, HLA-class I, CD58, CD155, and CD112 **(****Figures 4A****).** Although CD13⁺, BV-173 cells that were not killed by CD4^{IL-10} cells, were HLA-class I⁺CD58⁺ but expressed CD54 and CD112 at low levels. According to the role of HLA-class I in promoting CD4^{IL-10} cell-activation, although expressing CD54, CD58, CD155 or CD112, HLA-class I^{neg} K562 and Daudi cells were not killed **(****Figures 4A****).**

The inventors next determined whether CD4^{IL-10} cells can eliminate primary AML blasts **(Table 1).** As negative controls the inventors used primary ALL blasts. CD4^{IL-10} cells, generated from four different healthy donors, killed four out of eight primary AML blasts tested **(****Figure 4B****).** We performed correlation studies to define whether CD4^{IL-10} -mediated killing of primary AML blasts was associated with the expression of specific markers. Results showed that CD4^{IL-10}-mediated lysis correlated with the expression of CD13, of CD54, and of CD112 on AML blasts, but not with CD58 or CD155 **(****Figure 4C****).** Notably, CD4^{IL-10} cells did not eliminate Leu#7 that was CD13⁺CD54⁺ but CD112^{neg} **(****Figure 4C****).** Surprisingly, CD4^{IL-10} cells efficiently eliminate Leu#10 a primary ALL blasts that was CD13⁺ CD54⁺CD112⁺ **(****Figure 4B****).** The expression of CD13 is determinant for the anti-leukemic activity of CD4^{IL-10} cells, since two human multiple myeloma cell lines U266 and MM1S that were CD54⁺CD112⁺ but did not express CD13 were not killed **((**Figure 5A-B).Based on these data, we conclude that CD4^{IL-10} cells eliminate CD13⁺ leukemic cells and optimal CD4^{IL-10}-mediated killing requires stable CD54/LFA-1-mediated adhesion and CD112/CD226-mediated activation **(****Figure 6****).** CD13, CD54, and CD112 can be used as biomarkers for the identification of target of anti-leukemic effect of CD4^{IL-10} cells.

**CD^{IL-10} cells mediate anti-leukemic effects *in vivo.*** To test the anti-leukemic activity of CD4^{IL-10} cells *in vivo,* we used four different clinically-relevant humanized models: the subcutaneous myeloid sarcoma, the ALL-CM leukemia model ^{20,21}, the extramedullary myeloid tumor ²⁴, and the GvL/xeno-GvHD model of T-cell immunotherapy. The inventors developed the humanized model of subcutaneous myeloid sarcoma: NSG mice were sub-cutaneously injected with ALL-CM cells and three weeks later developed subcutaneous myeloid sarcoma (13.9±1.16 mm, mean±SEM, n=9, **Figure 7A****).** In this model, PBMC, CD4^{IL-10} or CD4^{GFP} cells were injected within the tumor at day three after ALL-CM infusion. Treatment with CD4^{IL-10} cells significantly delayed myeloid sarcoma growth **(****Figure 7A****).** Notably, in CD4^{IL-10}-injected mice no signs of tumor development were observed within the first 14 days, and on day 21 the tumor size in CD4^{IL-10} -treated mice was significantly lower than that of un-treated mice (7.6±0.9 mm, mean ± SEM, n=8; p≤0.001; **Figure 7A****).** As expected, mice injected with CD4^{GFP} cells developed myeloid sarcoma similarly to control untreated mice, whereas injection of allogeneic PBMC completely prevented tumor growth **(****Figure 7A****).**

The inventors next evaluated whether CD4^{IL-10} cells mediate anti-leukemic effects *in vivo* using the ALL-CM leukemia model of T-cell therapy in unconditioned NSG mice ^{21,22}. After ALL-CM cell infusion, NSG mice developed leukemia in four weeks **(****Figure 7B****).** In this system, injection of allogeneic PBMC, three days after ALL-CM challenge, delayed leukemia progression **(****Figure 7B****),** and after four weeks we detected a significantly lower proportion of circulating human blasts (on average 4.8%, p≤0.01) as compared to that control untreated mice (data not shown). Adoptive transfer of CD4^{IL-10} cells or CD4^{GFP} cells at day three after ALL-CM infusion did not delay leukemia development **(****Figure 7B****),** and at week four the percentage of circulating blasts was comparable to that observed in control untreated mice (data not shown).

Overall, we showed that CD4^{IL-10} cells delayed the subcutaneous myeloid sarcoma development, while they do not inhibit the leukemia growth. We postulated that in the model of ALL-CM leukemia CD4^{IL-10} cells do not co-localize with leukemic cells in the bone marrow. To test this hypothesis, we first investigated the expression of CXCR4 known to regulate the homing of human hematopoietic stem cells and myeloid leukemia in the bone marrow of humanized mice ²⁵⁻²⁷. In contrast to ALL-CM cells and PBMC, resting CD4^{IL-10} cells do not express significant levels of CXCR4 **(****Figure 8****).** We next investigated the *in vivo* localization of CD4^{IL-10} cells in NSG mice injected with ALL-CM and three days later with PBMC, CD4^{IL-10} or CD4^{GFP} cells. The frequency of circulating CD4^{IL-10} cells at day seven after transfer was on average 16.3% (±15.7%, mean±STD, n=7), and declined to 6.7% (±2.2%, mean±STD, n=3) at day fourteen **(****Figure 7C****).** Similar results were obtained in mice injected with CD4^{GFP} cells. In the bone marrow, CD4^{IL-10} cells were detected at very low frequency at day seven (0.4±0.5% mean±STD, n=7), and disappeared at day fourteen **(****Figure 7C****).** Moreover, at day seven, CD4^{IL-10} and CD4^{GFP} cells localized in the spleen and lung and their frequencies declined on day fourteen **(****Figure 7C****).** Finally, although at low frequency, CD4^{IL-10} cells were also identified in the liver at both time points analyzed **(****Figure 7C****).** In peripheral blood of mice treated with PBMC more than 70% and 30% of human T cells were found at day seven and fourteen, respectively **(****Figure 7C****).** T cells were also identified in the spleen, lung, liver, and in bone marrow of PBMC-injected mice at both time points **(****Figure 7C****).**

Since CD4^{IL-10} cells localize in the liver, we tested the anti-leukemic activity of CD4^{IL-10} cells in a model of THP-1 myeloid tumor ²⁴ **(****Figure 7D****).** In this model, PBMC were injected in NSG mice at day three after THP-1 cell infusion, whereas CD4^{IL-10} or CD4^{GFP} cells were infused two weeks after tumor injection, time in which the presence of THP-1 cells was evident in the liver (data not shown). Results demonstrated that adoptive transfer of CD4^{IL-10} cells and of PBMC inhibited in a comparable manner the ability of THP-1 cells to form liver nodules, whereas no difference in tumor development were observed between CD4^{GFP}-injected and control untreated mice **(****Figure 7D****).**

These findings indicate that despite the limited *in vivo* lifespan, CD4^{IL-10} cells mediate potent and specific anti-leukemic effects in peripheral tissues in different humanized models.

### Adoptive transfer of CD^{IL-10} cells prevents xeno-GvHD while spearing the GvL of allogeneic T cells.

The inventors next investigated the effects of CD4^{IL-10} cells on both GvL and xeno-GvHD mediated by allogeneic human T cells *in vivo.* To this end, we developed a humanized model of GvL/xeno-GvHD: NSG mice were sub-lethally irradiated, injected with ALL-CM cells, and three days later received allogeneic PBMC alone or in combination with CD4^{IL-10} cells **(****Figure 9A** and B). Since conditioning causes an increase in SDF-1 expression and secretion by stromal cells within murine bone marrow ²⁸, we analyzed the *in vivo* localization of CD4^{IL-10} cells in sub-lethally irradiated NSG mice injected with ALL-CM and three days later with PBMC, CD4^{IL-10} or CD4^{GFP} cells. The frequency of CD4^{IL-10} cells in the bone marrow at day seven after transfer resulted on average 22.3% (±9.6%, mean±SEM, n=9), and then declined to 0.3% (±0.2%, mean±SEM, n=11) at day fourteen **(****Figure 9A****).** The peripheral blood tested in parallel showed comparable results, with up to 34.3% (±8.2%, mean±SEM, n=17) of circulating CD4^{IL-10} cells at day seven, that then declined to 3.0% (±1.6%, mean±SEM, n=14) at day fourteen. Similar results were obtained in mice injected with CD4^{GFP} cells **(****Figure 9A****).** In the bone marrow of PBMC-injected mice 39.4% (±7.0%, mean±SEM, n=8) and 23.5% (±5.0%, mean±SEM, n=14) of human T cells were detected at day seven and fourteen, respectively. In the peripheral blood of PBMC-injected mice, human T cells were detected at both time points analyzed **(****Figure 9A****).** These data indicate that in conditioned NSG mice CD4^{IL-10} cells migrate in the bone marrow.

The inventors then tested the ability of CD4^{IL-10} cells to mediate anti-leukemic effect in conditioned NSG mice. Results showed that adoptive transfer of CD4^{IL-10} cells at day three after ALL-CM injection significantly delayed leukemia progression (P<0.05), whereas, treatment with CD4^{GFP} cells did not **(****Figure 9B****).** It should be noted that leukemia progression in CD4^{IL-10}-injected mice was not significantly different to that observed with PBMC-injected mice (*P*≤0.0001, **Figure 9B****).** However, PBMC-treated mice developed severe xeno-GvHD and died within twenty days **(****Figure 9B****).** Remarkably, co-transfer of CD4^{IL-10} cells and PBMC significantly delayed leukemia progression (P<0.05), with no sign of xeno-GvHD **(****Figure 9B****).** Overall, these findings demonstrate that adoptive transfer of CD4^{IL-10} cells prevents xeno-GvHD, while spearing the GvL mediated by allogeneic T cells.

Further, using the LV-IL-10 platform the inventors generated alloantigen-specific CD4^{IL-10} cells that kill myeloid leukemic cell lines *in vitro* in an antigen-independent manner. Alloantigen-specific LV-10 transduced T cells (allo)CD4^{IL-10} were generated by stimulation of naïve CD4⁺ T cells stimulated with allogeneic mature dendritic cells (allo-mDC) and transduction upon secondary stimulation (Figure 11A). Transduction efficiency of naïve CD4⁺ cells was on average 55% (Figure 11B). Allo-CD4^{IL-10} cells proliferated significantly less (p≤0.001, **Figure 12A****)** and produced significantly higher levels of IL-10 upon restimulation with allo-mDC, compared to allo-CD4^{ΔNGFR} cells (p≤0.0001, **Figure 12B****).** Conversely, allo-CD4^{IL-10} and allo-CD4^{ΔNGFR} cells showed similar low proliferative capacity in response to third party antigens, **(****Figure 12A****),** although allo-CD4^{IL-10} produced significantly higher levels of IL-10 (p≤0.001, **Figure 12B****).** Allo-CD4^{IL-10} but not allo-CD4^{ΔNGFR} cells efficiently suppressed the proliferation of allo-specific T cell lines activated with allo-mDC, but not with a third party mDC **(****Figure 12C****).** Allo-CD4^{IL-10} cells killed ALL-CM and U937, but not K562 leukemic cell lines, similar to what we observed with polyclonal CD4^{IL-10} cells **(****Figure 12D****).** These results show that enforced IL-10 expression in allo-specific CD4⁺ T cells promotes their conversion into Tr1-like cells able to kill myeloid cell lines.

To determine the role of HLA-class I expression on target cells in CD4^{IL-10}-mediated killing, we selectively deleted HLA-class I expression on ALL-CM and U937 cell lines by disrupting the β₂-microglobulin encoding gene. β2m-deficient (β2m^{-/-}) ALL-CM and U937 cell lines **(****Figure 13****),** were killed by CD4^{IL-10} cells at significantly lower levels compared to 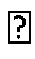 2m positive ALL-CM (p<0.001) and U937 (p<0.05) cell lines (Figure 14A). Moreover, we showed that the addition of a pan anti-HLA-class I (clone W6/32) mAb not inhibited CD4^{IL-10} -mediated killing of ALL-CM and U937 cell lines (p<0.05; **Figure 2D** **patent),** and prevented the degranulation of CD4^{IL-10} cells as shown by the significantly (p<0.05) lower frequency of GzB⁺CD107a⁺ cells in co-cultured of CD4^{IL-10} cells and ALL-CM or U937 cell lines in the presence of the W6/32 mAb (Figure 14B). Interestingly, addition of a pan anti-HLA class II mAb does not inhibit the the degranulation of CD4^{IL-10} cells co-cultures with either ALL-CM or U937 cell lines, indicating that CD4^{IL-10} cells do not require TCR-mediated activation to expert their cytolitic effects against myeloid target cells **(****Figure 15****).**

### Discussion

The Inventors previously showed that enforced expression of IL-10 confers a Tr1-like phenotype and function to human CD4⁺ T cells, including killing of myeloid cells ¹⁹. They now generate CD4^{IL-10} cells using a novel bidirectional LV encoding for human *IL-10* and *ΔNGFR,* as clinical grade marker gene. The inventors show that CD4^{IL-10} cells acquired the expression of CD18, CD2, and CD226, and the ability to secrete GzB. They provide evidences that CD4^{IL-10} cells kill leukemic cell lines *in vitro* and *in vivo,* and that this killing is specific for target cells, preferably myeloid cells. For the first time we establish that CD4^{IL-10} cells eliminate primary leukemic blasts. We demonstrate that the expression of HLA-class I on target myeloid cells is necessary but not sufficient for promoting CD4^{IL-10}-mediated cytotoxicity, which also requires stable CD54-mediated adhesion, and activation *via* CD226. CD13, CD54, and CD112 are biomarkers of CD4^{IL-10}-mediated killing of primary blasts. In humanized mouse models, CD4^{IL-10} cells mediate potent anti-leukemic effects and prevent xeno-GvHD without compromising the GvL mediated by allogeneic T cells.

Correlation between the expression of the marker CD13 and the ability of CD4^{IL-10} cells to eliminate primary blasts **(****Figure 4****)** indicates that CD13 expression determines the target specificity of CD4^{IL-10} cells. Furthermore, killing of primary blasts by CD4^{IL-10} cells correlates with CD54 expression **(****Figure 4****),** revealing the importance of stable adhesion between target and CD4^{IL-10} cells for effective cytolysis, as it was previously shown for Tr1 cells ⁶. CD54 on myeloid blasts allows stable and prolonged interaction with LFA-1 (CD18-CD11a) on CD4^{IL-10} cells, which is required for achieving the signaling threshold necessary to activate effector function that culminate with the secretion of lytic granules containing GzB. Nonetheless, the expression of CD54 is not sufficient to render CD13⁺ blasts target of CD4^{IL-10}-mediated lysis. The expression of CD112, one of the ligand of CD226 involved in Tr1-mediated cytolysis ⁶, is also required **(****Figure 4****).** The interaction between CD112 on myeloid leukemic blasts and CD226 on CD4^{IL-10} cells leads to their activation, resembling the mechanism previously described for NK-mediated lysis of AML blasts ²⁹. Being the downstream signaling of CD226 dependent on its association with LFA-1 ³⁰, we can conclude that CD112/CD226 interaction promotes the stabilization of CD4^{IL-10}- target cell conjugate that triggers CD4^{IL-10} cell degranulation. CD226-mediated activation of T cells can be inhibited by TIGIT ³¹, another receptor for CD112 and CD155 ³². TIGIT binds to CD155 with higher affinity that CD226 and inhibits the cytotoxicity and IFN-γ production of NK cells ^{33,34}. Of note, CD4^{IL-10} cells express TIGIT **(****Figure 10****);** nevertheless, the finding that CD155 is less express than CD112 on primary blasts ^{29,35} and that CD4^{IL-10}-mediated lysis does not correlate with the expression of CD155, suggests that activation of CD4^{IL-10} cells via CD226/CD112 interaction is dominant on the inhibition mediated by TIGIT/CD155. Based on our findings we find that the selective expression of CD13, CD54 and CD112 on leukemic blasts could be used as biomarker of anti-leukemic effect mediated by CD4^{IL-10} cells.

The inhibition of HLA class I recognition by neutralizing mAbs, or lack of HLA class I expression on target blasts prevents the lytic activity mediated by CD4^{IL-10} cells **(****Figure 2****),** as shown for Tr1 cells ⁶. In addition to CD226-mediated activation, CD4^{IL-10} cells need to be triggered *via* HLA-class I to release GzB and kill target cells. The ability of CD4^{IL-10} cells to eliminate myeloid leukemia in an alloAg-independent but HLA class I-dependent manner renders them an interesting tool to limit, and possibly to overcome, leukemia relapse caused by the lost of shared HLA alleles after haploidentical-HSCT, or matched unrelated HSCT ³⁶⁻³⁸.

CD4^{IL-10} cells mediate anti-leukemic effect in humanized murine models of myeloid tumors. This anti-tumor effect is strictly related to the co-localization of CD4^{IL-10} and leukemic cells. We show that CD4^{IL-10} cells display an effective anti-tumor activity when either locally injected within the myeloid sarcoma, or systemically injected in mice with liver-bearing myeloid tumors **(****Figure 7****).** Moreover, CD4^{IL-10} cell immunotherapy prevents leukemia development in conditioned humanized mice, in which a significant frequency of CD4^{IL-10} cells is present in the bone marrow as soon as seven days post-infusion **(****Figure 9****).** The anti-leukemic effect exerted within the bone marrow by CD4^{IL-10} cells is not achieved in unconditioned humanized mice **(****Figure 7****).** In the latter setting, CD4^{IL-10} cells are present in limited numbers and for a short period of time in the bone marrow, and therefore they do not control leukemia progression, which occurs at later time points. It should be noted that the limited lifespan of CD4^{IL-10} cells could be beneficial in case of immunotherapy, as it could avoid the risk of general immunosuppression. This is one of the important aspects to be considered in case of Treg-cell based immunotherapy.

CD4^{IL-10} cell immunotherapy prevents xeno-GvHD without hampering the anti-leukemic effect of allogeneic PBMC **(****Figure 9****).** Similarly, co-infusion of *in vitro* expanded or freshly isolated human CD4⁺CD25⁺ Tregs with allogeneic PBMC ³⁹ or conventional donor T cells ¹⁷ rescued humanized mice from leukemia and survived without xeno-GvHD. In the first study, human CD4⁺CD25⁺ Tregs have been show to migrate into the bone marrow, where they are converted into IL-17-producing T cells and lose the ability to suppress anti-tumor activity of human T cells ³⁹. Conversely, Martelli et al. ¹⁷ proposed that the failure of human CD4⁺CD25⁺ Tregs to home to the bone allows allogeneic T cells to control leukemia development. Our data support the hypothesis that CD4^{IL-10} cells behave differently from human CD4⁺CD25⁺ Tregs since they migrate in the bone marrow where they eliminate myeloid leukemia without limiting the GvL mediated by allogeneic PBMC.

Overall, the present data support the hypothesis that immunotherapy with CD4^{IL-10} cells can: i) mediate anti-leukemic effects, GvL; ii) mediate anti-tumor effect, GvT, iii) allow to maintain the GvL effect of allogeneic T cells; and ivi) maintain the ability to inhibit GvHD.

Moreover, the inventors showed that LV-hIL-10 can be used to convert allo-specific CD4+ T cells into allo-specific Tr1-like cells (allo-CD4^{IL-10} cells), which specifically kill myeloid target cells and suppress allo-specific T cell responses *in vitro.* Interestingly, the lack of HLA-class I on target cells, or the inhibition of HLA class I recognition by neutralizing mAbs, abrogate the CD4^{IL-10}-mediated killing *in vitro* and *in vivo,* suggesting that activation of CD4^{IL-10} cells through receptor/HLA class I interaction is necessary for GzB release and killing of target cells. Conversely, inhibition of HLA-class II does not impair CD4^{IL-10} cell activation and the elimination of target myeloid cells.

The present invention provides evidence for the use of CD4^{IL-10} cell immunotherapy after allo-HSCT for hematological malignancies aimed at inhibiting GvHD while allowing to maintain GvL. The expression of CD13, CD54 and HLA-I and optionally CD112 on tumor cells, in particular myeloid blasts allows patient selection and to design *ad hoc* therapeutic protocol.

Moreover the present invention provides evidences for the use of polyclonal CD4^{IL-10} cell or allo-specific immunotherapy for mediating GvT and providing GvL in the contest of tumor or allo-HSCT, respectively. Moreover, the finding that CD4^{IL-10} cells eliminate myeloid leukemia in a TCR-independent but HLA class I-dependent manner suggests their possible use to limit, and possibly to overcome, leukemia relapse caused by the loss of not-shared HLA alleles after allo-HSCT.

### References

1. Sakaguchi, S., Sakaguchi, N., Asano, M., Itoh, M. & Toda, M. J Immunol 155, 1151-1164 (1995).
2. Groux, H., et al. Nature 389, 737-742 (1997).
3. Gagliani, N., et al. Nat Med (2013).
4. Bacchetta, R., et al. J Exp Med 179, 493-502 (1994).
5. Roncarolo, M.G., et al. Immunol Rev 212, 28-50 (2006).
6. Magnani, C.F., et al. Eur J Immunol Apr 6. (2011).
7. Bacchetta, R., et al. Haematologica (2010).
8. Gregori, S., et al. Blood 116, 935-944 (2010).
9. Hoffmann, P., et al. J Exp Med 196, 389-399 (2002).
10. Edinger, M., et al. Nat Med 9, 1144-1150 (2003).
11. Cohen, J.L., et al. J Exp Med 196, 401-406 (2002).
12. Gregori, S., Goudy, K.S. & Roncarolo, M.G. Front Immunol 3, 30 (2012).
13. Trzonkowski, P., et al. Clin Immunol 133, 22-26 (2009).
14. Edinger, M. & Hoffmann, P. Curr Opin Immunol 23, 679-684 (2011).
15. Di lanni, M., et al. Blood 117, 3921-3928 (2011).
16. Brunstein, C.G., et al. IBlood (2010).
17. Martelli, M.F., et al. Blood (2014).
18. Bacchetta, R., et al. Front Immunol 5, 16 (2014).
19. Andolfi, G., et al. Mol Ther 20, 1778-1790 (2012).
20. De Palma, M. & Naldini, L. Methods Enzymol 346, 514-529 (2002).
21. Bondanza, A., et al. Blood 107, 1828-1836 (2006).
22. Hambach, L., et al. Leukemia 20, 371-374 (2006).
23. Pegoraro, L., et al. J Natl Cancer Inst 70, 447-453 (1983).
24. Casucci, M., et al. Blood 122, 3461-3472 (2013).
25. Dar, A., Kollet, O. & Lapidot, T. Exp Hematol 34, 967-975 (2006).
26. Tavor, S., et al. Cancer Res 64, 2817-2824 (2004).
27. Kollet, O., et al. Blood 97, 3283-3291 (2001).
28. Ponomaryov, T., et al. J Clin Invest 106, 1331-1339 (2000).
29. Pende, D., et al. Blood 105, 2066-2073 (2005).
30. Shibuya, K., et al. Immunity 11, 615-623 (1999).
31. Lozano, E., Dominguez-Villar, M., Kuchroo, V. & Hafler, D.A. J Immunol 188, 3869-3875 (2012).
32. Yu, X., et al. Nat Immunol 10, 48-57 (2009).
33. Georgiev, H., et al. Eur J Immunol 44, 307-308 (2014).
34. Stanietsky, N., et al. Eur J Immunol 43, 2138-2150 (2013).
35. Sanchez-Correa, B., et al. Immunol Cell Biol 90, 109-115 (2012).
36. Vago, L., et al. N Engl J Med 361, 478-488 (2009).
37. Villalobos, I.B., et al. Blood 115, 3158-3161 (2010).
38. Toffalori, C., et al. Blood 119, 4813-4815 (2012).
39. Guichelaar, T., et al. Clin Cancer Res 19, 1467-1475 (2013).
40. Bacchetta, R., et al. Blood, 45 (ASH Annual Meeting Abstract) (2009).
41. Bondanza A, et al. Blood.;117:6469-78 (2011).

## Claims

1. A human IL-10-expressing CD4⁺ T cell population for use in combination with allo-HSCT for the treatment of a hematological or solid tumor, wherein said cell population comprises cells that have been genetically modified as to constitutively overexpress human IL-10.

2. The CD4⁺ T cell population for use according to claim 1, wherein the cells express high levels of human IL-10 in respect to a reference cell.

3. The CD4⁺ T cell population for use according to any one of the preceding claims, wherein the CD4⁺ T cells are heterologous to the patient.

4. The CD4⁺ T cell population for use according to any one of the preceding claims, wherein the CD4⁺ T cells are heterologous to the allo-HSCT donor.

5. The CD4⁺ T cell population for use according to any one of the preceding claims, wherein the CD4⁺ T cells are not allo-specific.

6. The CD4⁺ T cell population for use according to any one of the preceding claims, wherein the CD4⁺ T cells inhibit GvHD.

7. The CD4⁺ T cell population for use according to any one of the preceding claims, wherein the CD4⁺ T cells do not inhibit Graft versus Tumor (GvT) activity and/or Graft versus Leukemia (GvL) activity of the allo-HSCT graft.

8. The CD4⁺ T cell population for use according to any one of the preceding claims, wherein the hematological or solid tumor is selected from Adrenal Cancer, Anal Cancer, Bile Duct Cancer, Bladder Cancer, Bone Cancer, Brain/CNS Tumors In Adults, Brain/CNS Tumors In Children, Breast Cancer, Breast Cancer In Men, Cancer of Unknown Primary, Castleman Disease, Cervical Cancer, Colon/Rectum Cancer, Endometrial Cancer, Esophagus Cancer, Ewing Family Of Tumors, Eye Cancer, Gallbladder Cancer, Gastrointestinal Carcinoid Tumors, Gastrointestinal Stromal Tumor (GIST), Gestational Trophoblastic Disease, Hodgkin Disease, Kaposi Sarcoma, Kidney Cancer, Laryngeal and Hypopharyngeal Cancer, Leukemia, Acute Lymphocytic (ALL), Acute Myeloid (AML, including myeloid sarcoma and leukemia cutis), Chronic Lymphocytic (CLL), Chronic Myeloid (CML) Leukemia, Chronic Myelomonocytic (CMML), Leukemia in Children, Liver Cancer, Lung Cancer, Lung Cancer with Non-Small Cell, Lung Cancer with Small Cell, Lung Carcinoid Tumor, Lymphoma, Lymphoma of the Skin, Malignant Mesothelioma, Multiple Myeloma, Myelodysplastic Syndrome, Nasal Cavity and Paranasal Sinus Cancer, Nasopharyngeal Cancer, Neuroblastoma, Non-Hodgkin Lymphoma, Non- Hodgkin Lymphoma In Children, Oral Cavity and Oropharyngeal Cancer, Osteosarcoma, Ovarian Cancer, Pancreatic Cancer, Penile Cancer, Pituitary Tumors, Prostate Cancer, Retinoblastoma, Rhabdomyosarcoma, Salivary Gland Cancer, Sarcoma -Adult Soft Tissue Cancer, Skin Cancer, Skin Cancer - Basal and Squamous Cell, Skin Cancer - Melanoma, Skin Cancer - Merkel Cell, Small Intestine Cancer, Stomach Cancer, Testicular Cancer, Thymus Cancer, Thyroid Cancer, Uterine Sarcoma, Vaginal Cancer, Vulvar Cancer, Waldenström's Macroglobulinemia, and Wilms Tumor.

9. The CD4⁺ T cell population for use according to any one of the preceding claims, wherein said hematological or solid tumor expresses CD 13 and HLA-class 1.

10. The CD4⁺ T cell population for use according to claim 9, wherein the hematological or solid tumor is a myeloid malignancy.

11. The CD4⁺ T cell population for use according to claim 10, wherein the myeloid malignancy is Acute Myeloid Leukemia (AML), including myeloid sarcoma and leukemia cutis, Chronic Myeloid (CML) Leukemia, or Chronic Myelomonocytic (CMML).

12. The CD4⁺ T cell population for use according to any one of claims 9 to 11, wherein the CD4⁺ T cells are capable of eliminating primary leukemic blasts.

13. The CD4⁺ T cell population for use according to any one of claims 9 to 12, wherein the CD4+ T cells are capable of treating leukemia relapse.

14. The CD4⁺ T cell population for use according to any of the preceding claims, wherein the allo-HSCT is haploidentical-HSCT or matched unrelated HSCT.

15. A composition comprising a population of CD4⁺ T cells as defined in any one of the preceding claims and proper excipients for use in the treatment of a hematological or solid tumor.
